# EUROPÄISCHE PATENTSCHRIFT

(11) **EP 3 847 153 B1**
(45) Veröffentlichungstag und Bekanntmachung des Hinweises auf die Patenterteilung: **01.05.2024**
(21) Anmeldenummer: 19762399.4
(22) Anmeldetag: 05.09.2019
(51) Int. Cl.: C07C 69/704, A61K 8/37, A61Q 19/00, A61K 8/06, A23L 29/10

(54) **ECO-EMULGATOR**
ECO EMULSIFIER
ÉCO-ÉMULSIFIANT

(30) Priorität: 05.09.2018 WO PCT/EP2018/073899
(43) Veröffentlichungstag der Anmeldung: 14.07.2021
(73) Patentinhaber: Symrise AG, 37603 Holzminden (DE)
(72) Erfinder: SCHMAUS, Gerhard, 37671 Höxter-Bosseborn (DE); BUGDAHN, Nikolas, 37603 Holzminden (DE); LANGE, Sabine, 37603 Holzminden (DE); DROEGE, Dietmar, 31868 Ottenstein (DE); SCHADE, Vanessa, 37603 Holzminden (DE)
(74) Vertreter: Eisenführ Speiser
(86) Internationale Anmeldenummer: PCT/EP2019/073644
(87) Internationale Veröffentlichungsnummer: WO 2020/049077

(56) Entgegenhaltungen:
- EP-A1- 2 111 850
- WO-A2-2004/112731
- CN-A- 105 541 614
- FR-M- 3 000
- US-A- 2 813 032
- US-A- 4 071 544
- US-A1- 2011 273 646
- PROSPERIO G ET AL: "Neuere essbare O/W-Emulgator-Mischungen", RIECHSTOFFE AROMEN KOSMETICA (RAK), FACHVERLAG V. FRANKENSTEIN. ESCHERSHAUSEN, DE, Bd. 28, Nr. 1, 1. Januar 1978 (1978-01-01) , Seiten 8-12, XP002112591,

## Beschreibung

Die vorliegende Erfindung betrifft primär neue Emulgatoren umfassend oder bestehend aus einer Mischung aus Verbindungen der Formel (I) 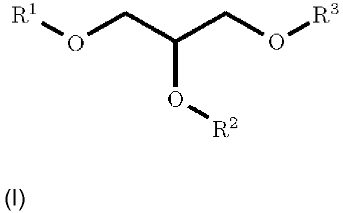 wie hierin beschrieben (für die Bedeutung der Reste R¹, R² und R³ siehe weiter unten), einem oder mehreren unterschiedlichen Salz(en) der Verbindung(en) der Formel (I) wie hierin beschrieben oder einer oder mehreren unterschiedlichen Verbindung(en) der Formel (I) wie hierin beschrieben und einem oder mehreren unterschiedlichen Salz(en) der Verbindung(en) der Formel (I) wie hierin beschrieben. Im Folgenden bezeichnet "Verbindung der Formel (I)" sowie ein Salz einer solchen Verbindung bzw. eine Mehrzahl solcher Verbindungen oder Salze oder Mischungen daraus eine Verbindung der Formel (I) "wie hierin beschrieben" bzw. ein Salz einer solchen Verbindung oder eine Mehrzahl solcher Verbindungen oder Salze oder Mischungen daraus.

Zudem betrifft die vorliegende Erfindung Emulgator-Vorprodukte umfassend oder bestehend aus einer oder mehreren Verbindung(en) der Formel (I) und/oder einem oder mehreren Salz(en) der Verbindung(en) der Formel (I).

Ein weiterer Aspekt der vorliegenden Erfindung betrifft ein Verfahren zur Herstellung des erfindungsgemäßen Emulgators und des erfindungsgemäßen Emulgator-Vorprodukts.

Des Weiteren betrifft die vorliegende Erfindung die Verwendung erfindungsgemäßer Mischungen aus Verbindung(en) der Formel (I) als Emulgator.

Zudem betrifft die vorliegende Erfindung auf einem oder mehreren erfindungsgemäßen Emulgator-Vorprodukt(en) und/oder einem oder mehreren erfindungsgemäßen Emulgator(en) basierende Emulsionen umfassend oder bestehend aus einer wässrigen Phase und einer Öl-Phase und ein Verfahren zur Herstellung der erfindungsgemäßen Emulsionen.

Ein weiterer Aspekt der vorliegenden Erfindung betrifft die Verwendung einer oder mehrerer erfindungsgemäßer Emulsionen in der Reinigung dienenden Zubereitungen, kosmetischen oder pharmazeutischen, vorzugsweise dermatologischen, Zubereitungen, dem Genuss oder der Ernährung dienenden Zubereitungen oder Halbfertigwaren, umfassend ein erfindungsgemäßes oder mehrere erfindungsgemäße Emulgator-Vorprodukt(e) und/oder einen oder mehrere erfindungsgemäße Emulgator(en) und/oder eine oder mehrere erfindungsgemäße Emulsion(en) sowie diese erfindungsgemäßen Zubereitungen.

Weitere Aspekte der vorliegenden Erfindung sowie besonders geeignete Ausgestaltungen ergeben sich aus der folgenden Beschreibung, den Beispielen sowie den beigefügten Patentansprüchen.

Emulgatoren basierend auf natürlichen Fetten/Ölen und Citronensäure sind seit langem bekannt. Sie werden in der Lebensmittelindustrie als Emulgatoren, Komplexbildner (unter anderem zur Unterstützung der Wirkung von Antioxidantien) und Trägerstoffe mit der Bezeichnung E472c (Citronensäureester von Mono- und Diglyceriden von Speisefettsäuren bzw. Citrem) verwendet und sind unter anderem in Kuchen, Keksen, Blätterteiggebäck, Brot, Wurstwaren, Speiseeis und Desserts, Süßwaren und Backfetten zu finden (Schuster, G., et al., Emulgatoren für Lebensmittel, Berlin, Heidelberg, New York, Tokyo: Springer 1985, S. 107-114).

Emulgatoren sind Hilfsmittel zur Herstellung und zur Stabilisierung von Emulsionen, die im engeren Sinne als grenzflächenaktive Stoffe bzw. Tenside bezeichnet werden können und in der Regel als ölige bis wachsartige, aber auch pulverförmige Stoffe vorliegen.

Emulgatoren setzen die Grenzflächenspannung zwischen den Phasen von Emulsionen herab und bewirken eine Stabilisierung der gebildeten Emulsion. Strukturelles Kennzeichen von Emulgatoren ist ihr amphiphiler Molekülaufbau. Ein Molekül einer solchen Verbindung besitzt wenigstens eine Gruppe mit Affinität zu Substanzen starker Polarität und wenigstens eine Gruppe mit Affinität zu unpolaren Substanzen.

Emulsionen sind disperse Systeme von zwei oder mehreren miteinander nicht mischbaren Flüssigkeiten bzw. nicht mischbaren flüssigen Phasen. Die eine der flüssigen Phasen bildet dabei das Dispersionsmittel (auch: äußere, kontinuierliche oder zusammenhängende Phase), in dem die andere Phase (auch: innere oder disperse Phase) in Form feiner Tröpfchen verteilt ist. Der Teilchendurchmesser der Teilchen, die vorzugsweise in einer idealisierten Betrachtungswese als sphärisch angenommen werden oder deren Größe als die Größe einer äquivalenten Kugel selben Durchmessers angegeben wird ("Äquivalent-Kugel"), schwankt dabei. Die meisten Emulsionen zeigen uneinheitliche Teilchengröße und sind polydispers. Die meisten natürlichen und technischen Emulsionen bestehen aus Wasser und Öl oder Fett als nicht mischbare Phasen. Eine O/W-Emulsion bzw. Öl-in-Wasser-Emulsion ist ein Fett-Wasser-Gemisch, dessen kontinuierliche Phase wässrig ist. Entsprechend ist ein O/W-Emulgator ein Emulgator, der eine O/W-Emulsion stabilisiert bzw. zur ihrer Stabilität beiträgt.

EP 1 641 904 B1 offenbart einen O/W-Emulgator enthaltend 70 bis 90 Gew.-% Glyceryloleat Citrat und 10 bis 30 Gew.-% eines Viskositätsmodifizierers, der es erlaubt, stabile, kaltherstellbare und dünnflüssige, insbesondere sprühbare, O/W-Emulsionen herzustellen.

JP 2012 031250 A und US 2011/0273646 A1 offenbaren Glycerylolcitrat-Fettsäureester von Caprylsäure, Stearinsäure und Ölsäure als Bestandteil zur Herstellung von schützenden Filmen für optische Polarisatoren, insbesondere von optischen Polarisatoren in Flüssigkristall-basierten Liquid Crystal Display (LCD)-Geräten mit dem Ziel, besonders gut gegen Temperatur und Feuchtigkeit beständige LCD-Geräte bereitzustellen.

CN105541614 A offenbart Glycerolcitrat-Fettsäureester von Myristinsäure, Palmitinsäure und Stearinsäure. Die Verbindungen sind als O/W Emulgator für Bereiche wie Kosmetik, Lebensmittel usw. zu verwenden. Die Verbindungen sind dadurch enthalten, dass Glycerol mit Fettsäuren umgesetzt werden, und Citronensäure dazugegeben. US4071544 A1 offenbart ein Verfahren zur Herstellung von Estern von Citronensäure mit Fettsäure-Glyceriden. Die Verbindungen finden in der Kosmetik, Pharma oder Lebensmittelindustrie Verwendung. Als Fettsäurepartialglyceride werden die aus Myristinsäure, Laurinsäure, Caprinsäure für geeignet erklärt.

Trotz einer Vielzahl bekannter Emulgatoren besteht unter anderem in der Lebensmittel verarbeitenden Industrie auch weiterhin ein Bedarf an neuen Emulgatoren, die über ihre primären Eigenschaften als Lösungsvermittler zwischen nicht mischbaren Phasen hinaus vorzugsweise zusätzliche positive Eigenschaften, wie vernachlässigbare oder keine Toxizität und/oder (erhöhte) Hautverträglichkeit bzw. Schleimhautverträglichkeit besitzen und/oder verbesserte Eigenschaften im Sinne erhöhter Emulgierbarkeit (bei geringerem Verbrauch) und/oder einem breiteren Einsatzgebiet mit sich bringen.

Die primäre Aufgabe der vorliegenden Erfindung war es, neue Emulgatoren bereitzustellen, vorzugsweise solche, die sich gegenüber dem Stand der Technik durch zusätzliche verbesserte Eigenschaften, z.B. im Sinne erhöhter Emulgierbarkeit (bei geringerem Verbrauch) und/oder einem breiteren Einsatzgebiet und/oder der Eignung zur Anreicherung des Emulgators sowie besonders bevorzugt durch vernachlässigbare oder keine Toxizität, und/oder (erhöhte) Hautverträglichkeit bzw. (erhöhte) Schleimhautverträglichkeit auszeichnen.

Weitere Aufgabenstellungen, die der vorliegenden Erfindung zu Grunde liegen, ergeben sich aus den nachfolgenden Ausführungen und den beigefügten Patentansprüchen.

Die vorangehend genannte primäre Aufgabe der vorliegenden Erfindung wird erfindungsgemäß gelöst durch einen Emulgator umfassend oder bestehend aus einer Mischung aus Verbindungen der Formel (I) 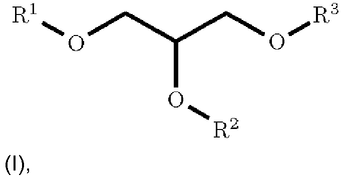 wobei jeweils gilt:
R¹, R² und R³ bedeuten jeweils unabhängig voneinander
   (i) ein Wasserstoffatom,
   (ii) einen durch eine Ester-Bindung an das Glycerol-Rückgrat gebundenen Fettsäurerest, oder
   (iii) einen durch eine Ester-Bindung an das Glycerol-Rückgrat gebundenen Citronensäurerest
   mit der Maßgabe, dass jeweils höchstens einer der Reste R¹, R² und R³ ein durch eine Ester-Bindung an das Glycerol-Rückgrat gebundener Citronensäurerest (iii) ist, einem oder mehreren unterschiedlichen Salz(en) der Verbindung(en) der Formel (I) oder einer oder mehreren unterschiedlichen Verbindung(en) der Formel (I) und einem oder mehreren unterschiedlichen Salz(en) der Verbindung(en) der Formel (I),
wobei der Anteil der Gesamtmenge an Verbindungen der Formel (I) und Salzen der Verbindung der Formel (I) mit einem oder zwei durch eine Ester-Bindung an das Glycerol-Rückgrat gebundenen Fettsäurerest(en) (ii) höher als 40 Gew.-%, höher als 50 Gew.-%, höher als 60 Gew.-%, höher als 70 Gew.-% höher als 80 Gew.-% , höher als 90 Gew.-% oder höher als 95 Gew.-% ist, bezogen auf das Gesamtgewicht des Emulgators,
wobei der Anteil der Gesamtmenge an Verbindungen der Formel (I) und Salzen der Verbindungen der Formel (I) mit genau einem durch eine Ester-Bindung an das Glycerol-Rückgrat gebundenen Citronensäurerest (iii) und mindestens einem durch eine Ester-Bindung an das Glycerol-Rückgrat gebundene Fettsäurerest (ii) höher als 20 Gew.-%, höher als 21 Gew.-%, höher als 22 Gew.-%, höher als 23 Gew.-%, höher als 24 Gew.-% oder als höher als 25 Gew.-% ist, bezogen auf das Gesamtgewicht des Emulgators und,
wobei der Anteil der Gesamtmenge an Verbindungen der Formel (I) und/oder einem oder mehrerer Salz(en) der Verbindung(en) der Formel (I) mit einem oder mehreren durch eine bzw. mehrere Ester-Bindung(en) an das Glycerol-Rückgrat gebundenen Fettsäurerest(en) (ii), bei denen der bzw. einer, mehrere oder sämtliche Fettsäurerest(e) (ii) unabhängig voneinander ausgewählt ist bzw. sind aus der Gruppe, bestehend aus dem Fettsäurerest der Capronsäure, dem Fettsäurerest der Caprylsäure, dem Fettsäurerest der Caprinsäure, dem Fettsäurerest der Laurinsäure und dem Fettsäurerest der Myristinsäure, höher als 55 Gew.-%, vorzugsweise höher als 70 Gew.-%oder höher als 75 Gew.-%, besonders bevorzugt höher als 90 Gew.-% ist, bezogen auf das Gesamtgewicht der Verbindungen der Formel (I), und
der Anteil der Gesamtmenge an Verbindungen der Formel (I) und/oder einem oder mehrerer Salz(en) der Verbindung(en) der Formel (I) mit einem oder mehreren durch eine bzw. mehrere Ester-Bindung(en) an das Glycerol-Rückgrat gebundenen Fettsäurerest (ii), bei denen der bzw. einer, mehrere oder sämtliche Fettsäurerest(e) (ii) unabhängig voneinander ausgewählt ist bzw. sind aus der Gruppe, bestehend aus dem Fettsäurerest der Laurinsäure und dem Fettsäurerest der Myristinsäure, höher als 50 Gew.-%, vorzugsweise höher als 55 Gew.-%, besonders bevorzugt höher als 60 Gew.-% ist, bezogen auf das Gesamtgewicht der Verbindungen der Formel (I), und
wobei für eine oder mehrere Verbindungen der Formel (I) gilt, dass der bzw. die Fettsäurerest(e) (ii) unabhängig voneinander ausgewählt ist bzw. sind aus der Gruppe, bestehend aus dem Fettsäurerest der Palmitinsäure, dem Fettsäurerest der Palmitoleinsäure, dem Fettsäurerest der Stearinsäure, dem Fettsäurerest der Ölsäure, dem Fettsäurerest der Linolsäure und dem Fettsäurerest der Linolensäure, niedriger als 40 Gew.-%, vorzugsweise niedriger als 35 Gew.-%, besonders bevorzugt niedriger als 30 Gew.-% ist, bezogen auf das Gesamtgewicht der Verbindungen der Formel (I).

In einer bevorzugten Ausführungsform des Emulgators umfasst die Mischung aus Verbindungen der Formel (I) 5 - 20 % Verbindungen der Formel (I) mit genau einem Fettsäurerest und maximal einem Citronensäurerest und 20 - 50 % Verbindungen der Formel (I) mit genau zwei Fettsäureresten und maximal einem Citronensäurerest und 25 - 60 % Verbindungen der Formel (I) mit genau drei Fettsäureresten.

Die erfindungsgemäß zu verwendenden Verbindungen der Formel (I) besitzen einen amphiphilen Molekül-Aufbau.

Ein "Fettsäurerest" im Sinne der vorliegenden Erfindung (s. hierzu z.B. die Bedeutung der Reste R¹, R² und R³ wie hierin beschrieben; Alternative (ii)) ist zu verstehen als ein Strukturbaustein, für den folgende Formel gilt: 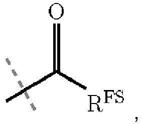 wobei die gestrichelte Linie die Bindung markiert, die jeweils einen der Reste R¹, R² oder R³ unabhängig voneinander unter Berücksichtigung der vorausgehenden Ausführungen und Maßgaben hinsichtlich der Verbindungen der Formel (I) mit jeweils einem der Sauerstoffatome, die in den Verbindungen der Formel (I) mit "O" gekennzeichnet sind, verknüpft und wobei R^{FS} ein entsprechend der dem Fachmann bekannten Nomenklatur ein univalentes Radikal ausgewählt aus der Gruppe, bestehend aus Alkylradikalen, Alkadienylradikalen und Alkatrienylradikalen bedeutet.

"Citronensäure" bzw. der davon abgeleitete "Citronensäurerest" im Sinne der Erfindung umfasst Citronensäure (2-Hydroxypropan-1,2,3-tricarbonsäure, insbesondere CAS: 77-92-9 bzw. InChlKey: KRKNYBCHXYNGOX-UHFFFAOYSA-N) bzw. den davon abgeleiteten Rest sowie deren Diastereomere bzw. die davon abgeleiteten Reste und deren Enantiomere bzw. die davon abgeleiteten Reste, insbesondere Isocitronensäure (3-Carboxy-2-hydroxy-pentan-1,5-disäure, insbesondere InChlKey: ODBLHEXUDAPZAU-FONMRSAGSA-N) bzw. den davon abgeleiteten Rest sowie deren Enantiomere bzw. die davon abgeleiteten Reste.

Ein durch eine Ester-Bindung gebundener Citronensäurerest im Sinne der vorliegenden Erfindung (s. hierzu z.B. die Bedeutung der Reste R¹, R² und R³ wie hierin beschrieben; Alternative (iii)) ist, hier beispielsweise für Citronensäure gezeigt, zu verstehen als ein Strukturbaustein, für den eine der folgenden Formeln (iii-a) oder (iii-b), gilt: 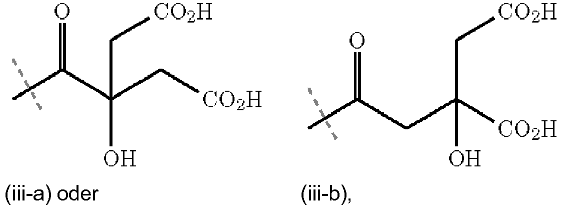 wobei die gestrichelte Linie die Bindung markiert, die jeweils einen der Reste R¹, R² oder R³ unabhängig voneinander unter Berücksichtigung der vorausgehenden Ausführungen und Maßgaben hinsichtlich der Verbindungen der Formel (I) mit jeweils einem der Sauerstoffatome, die in den Verbindungen der Formel (I) mit "O" gekennzeichnet sind, verknüpft.

Ein "Salz" der Verbindungen der Formel (I) im Sinne der vorliegenden Erfindung ist vorzugsweise ein Alkali- oder Erdalkalisalz von (teilweise) deprotonierten Verbindungen der Formel (I) an den, falls in den Verbindungen der Formel (I) vorhandenen, OH- oder COOH-Gruppen.

Ein erfindungsgemäßer Emulgator wie hierin beschrieben ist nicht aus dem Stand der Technik bekannt. In der Patentschrift EP 1 641 904 B1 beispielsweise wird nicht auf die Möglichkeit der Verwendung anderer als der darin beschriebenen, insbesondere kürzerkettiger, Fettsäurereste als Bestandteile der Glycerylolcitrat-Fettsäureester eingegangen. Stattdessen wird lediglich der positive Effekt von Capryl- bzw. Caprin-Triglyceriden als Viskositätsmodifizierer sowohl auf die Stabilität des O/W-Emulgators als auch auf die damit zubereiteten Emulsionen beschrieben. Die Dokumente JP 2012 031250 A und US 2011/0273646 A1 beispielsweise liefern ebenfalls keinen Hinweis auf die Verwendung weiterer als der darin genannten kürzerkettigen Fettsäurereste in den offenbarten Glycerylolcitrat-Fettsäureestern.

Als "kürzerkettige Fettsäurereste" im Sinne der vorliegenden Erfindung sind vorzugsweise Reste der Fettsäuren, wie im Folgenden definiert zu verstehen. Vorzugsweise sind kürzerkettige Fettsäurereste Reste ausgewählt aus der Gruppe, bestehend aus den zugehörigen Resten der Fettsäuren mit 6 bis 14 Kohlenstoffatomen, insbesondere Capronsäure, Caprylsäure, Caprinsäure, Laurinsäure und Myristinsäure. Besonders bevorzugt sind kürzerkettige Fettsäurereste Reste ausgewählt aus der Gruppe, bestehend aus den zugehörigen Resten der Fettsäuren mit 12 bis 14 Kohlenstoffatomen, insbesondere Laurinsäure und Myristinsäure. "Längerkettige Fettsäurereste" im Sinne der vorliegenden Erfindung sind vorzugsweise Reste der Fettsäuren ausgewählt aus der Gruppe, bestehend aus den zugehörigen Resten der Fettsäuren mit mehr als 14 Kohlenstoffatomen, insbesondere Palmitinsäure, Palmitoleinsäure, Stearinsäure, Ölsäure und Linolensäure.

Der "Anteil der Gesamtmenge an Verbindungen der Formel (I) und Salzen der Verbindungen der Formel (I) bezogen auf das Gesamtgewicht des Emulgators" wird vorzugsweise durch Vergleich von Massenspektren mit Referenzspektren unter Berücksichtigung der Elutionsposition und der UV-Spektren ermittelt bzw. bestimmt. Durch hochaufgelöste time of flight (TOF)-Massenspektren kann die Molmasse der Verbindung und gegebenenfalls eine Summenformel ermittelt werden. Die Probe wird flüssigchromatographisch aufgetrennt und mit Hilfe der Massenspektrometrie (MS) detektiert. Das erzeugte Massenspektrum ermöglicht eine Identifizierung der Einzelkomponenten. Dabei ist es möglich, zur weiteren Identifizierung ein MS-Spektrum von der Substanz zu erzeugen, sowie eine Detektion über Licht- bzw. Laserlichtstreuung durchzuführen. Alternativ ist auch eine gravimetrische Analyse anwendbar. Geringere Abweichungen bei der Bestimmung der Gew.-%-Angaben, z.B. durch unterschiedliche Messmethoden, sind im Rahmen der vorliegenden Erfindung hinnehmbar und für die Ausführbarkeit der hierin beschriebenen Erfindungsgegenstände nicht relevant.

Selbiges wie im vorangehenden Absatz gilt für Verbindungen mit genau zwei jeweils unabhängig voneinander durch Ester- Bindungen an das Glycerol-Rückgrat gebundene Citronensäurereste (iii) R¹, R² und R³.

Im Sinne der vorliegenden Erfindung sind gemäß einer Ausgestaltung Emulgatoren, bei denen der Anteil der Gesamtmenge an Verbindungen der Formel (I) und Salzen der Verbindungen der Formel (I) mit genau einem durch eine Ester-Bindung an das Glycerol-Rückgrat gebundenen Citronensäurerest (iii) höher als 85 Gew.-%, besonders bevorzugt bei denen selbiger Anteil höher als 95 Gew.-% ist, bezogen auf das Gesamtgewicht des Emulgators, bevorzugt.

In einer weiteren Ausführungsform der vorliegenden Erfindung handelt es sich um einen Emulgator umfassend oder bestehend aus einer oder mehreren unterschiedlichen Verbindung(en) der Formel (I) 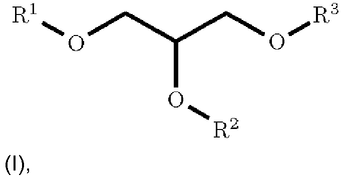 wobei jeweils gilt:
R¹, R² und R³ bedeuten jeweils unabhängig voneinander
   (i) ein Wasserstoffatom,
   (ii) einen durch eine Ester-Bindung an das Glycerol-Rückgrat gebundenen Fettsäurerest, oder
   (iii) einen durch eine Ester-Bindung an das Glycerol-Rückgrat gebundenen Citronensäurerest
   mit der Maßgabe, dass jeweils höchstens einer der Reste R¹, R² und R³ ein durch eine Ester-Bindung an das Glycerol-Rückgrat gebundener Citronensäurerest (iii) ist, einem oder mehreren unterschiedlichen Salz(en) der Verbindung(en) der Formel (I) oder einer oder mehreren unterschiedlichen Verbindung(en) der Formel (I) und einem oder mehreren unterschiedlichen Salz(en) der Verbindung(en) der Formel (I),
wobei der Anteil der Gesamtmenge an Verbindungen der Formel (I) und Salzen der Verbindungen der Formel (I) mit genau einem durch eine Ester-Bindung an das Glycerol-Rückgrat gebundenen Citronensäurerest (iii) höher als 20 Gew.-%, höher als 21 Gew.-%, höher als 22 Gew.-%, höher als 23 Gew.-%, höher als 24 Gew.-% oder als höher als 25 Gew.-% ist, bezogen auf das Gesamtgewicht des Emulgators.

In einer weiteren bevorzugten Ausführungsform des erfindungsgemäßen Emulgators, besteht die Mischung aus Verbindungen der Formel (I) aus 5 - 20 % Verbindungen der Formel (I) mit genau einem Fettsäurerest und/oder genau einem Citronensäurerest und aus 20 - 50 % Verbindungen der Formel (I) mit genau zwei Fettsäureresten und/oder genau zwei Fettsäureresten und genau einem Citronensäurerest und 25 - 50 % Verbindungen der Formel (I) mit genau drei Fettsäureresten und dem Rest ergänzend zu 100 % aus Verbindungen der Formel (I) mit genau drei Wasserstoffatomen und/oder Zitronensäure.

In einer Ausführungsform des erfindungsgemäßen Emulgators handelt es sich um eine Mischung, die aus einer oder mehreren Verbindung(en) der Formel (I) besteht oder solche umfasst, wobei der bzw. die Fettsäurerest(e) (ii) entsprechend der vorausgehenden Ausführungen und Maßgaben hinsichtlich der Verbindungen der Formel (I) unabhängig voneinander ausgewählt ist bzw. sind aus der Gruppe, bestehend aus dem Fettsäurerest der Capronsäure mit der Formel (ii-a): 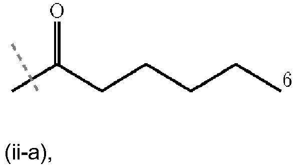 dem Fettsäurerest der Caprylsäure mit der Formel (ii-b): 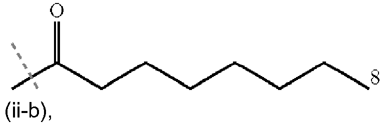 dem Fettsäurerest der Caprinsäure mit der Formel (ii-c): 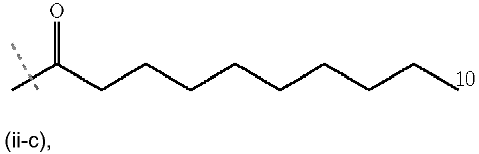 dem Fettsäurerest der Laurinsäure mit der Formel (ii-d): 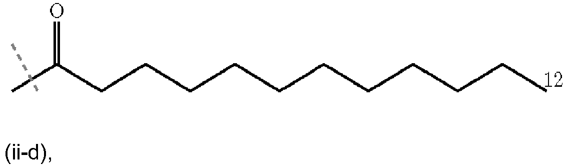 dem Fettsäurerest der Myristinsäure mit der Formel (ii-e): 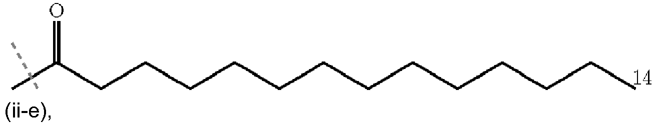 dem Fettsäurerest der Palmitinsäure mit der Formel (ii-f): 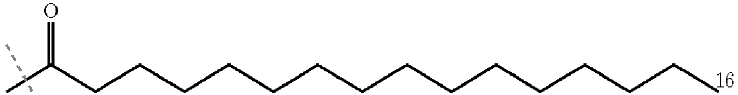 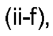 dem Fettsäurerest der Palmitoleinsäure mit der Formel (ii-g): 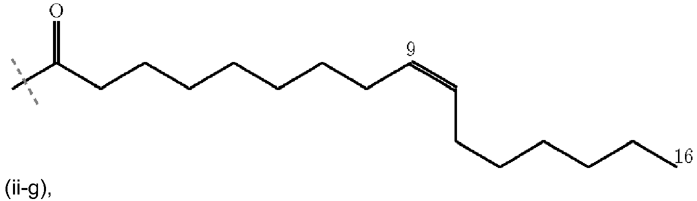 dem Fettsäurerest der Stearinsäure mit der Formel (ii-h) 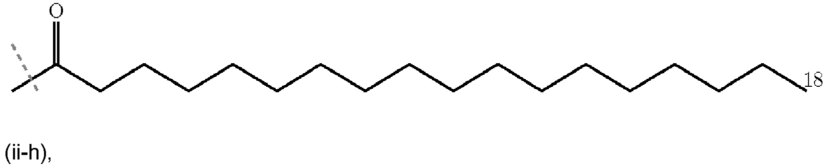 dem Fettsäurerest der Ölsäure mit der Formel (ii-i): 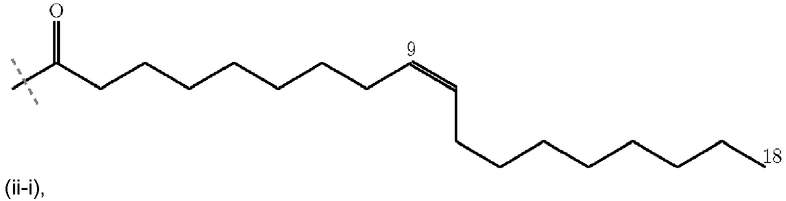 dem Fettsäurerest der Linolsäure mit der Formel (ii-j): 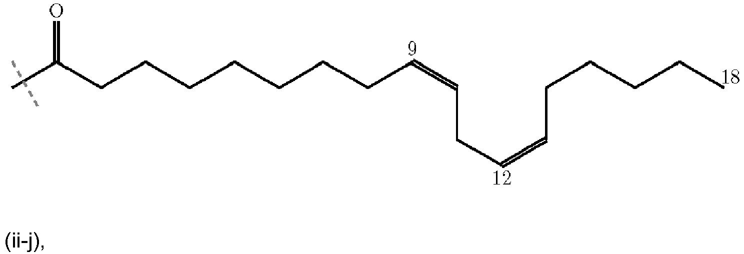 und dem Fettsäurerest der Linolensäure mit der Formel (ii-k): 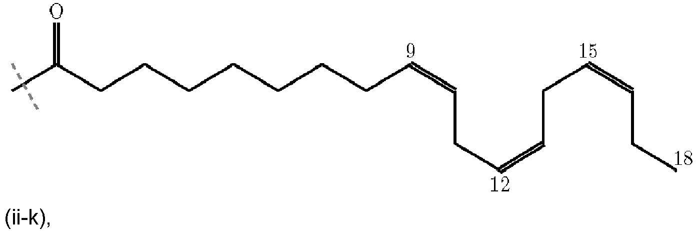 vorzugsweise aus der Gruppe, bestehend aus dem Fettsäurerest der Laurinsäure und dem Fettsäurerest der Myristinsäure, wobei die gestrichelte Linie der Fettsäurereste (ii-a) bis (iik) die Bindung markiert, die jeweils einen der Reste R¹, R² oder R³ unabhängig voneinander unter Berücksichtigung der vorausgehenden Ausführungen und Maßgaben hinsichtlich der Verbindungen der Formel (I) mit jeweils einem der Sauerstoffatome, die in den Verbindungen der Formel (I) mit "O" gekennzeichnet sind, verknüpft.

Im Falle eventueller Abweichungen zwischen einer gezeigten Strukturformel und dem genannten Namen der Verbindung bzw. des jeweiligen Strukturelements bzw. nicht vollumfänglich konventionsgemäßer Bezeichnung gilt die jeweilige angegebene Strukturformel bzw. das jeweilige Strukturelement.

Die vorangehend beschriebenen Fettsäurereste (ii-a) bis (ii-k) als Bestandteile, also einer oder mehrere der Reste R¹, R² und R³, der erfindungsgemäßen Emulgatoren weisen vorteilhafte lösungsvermittelnde Eigenschaften gegenüber bzw. stärker emulgierende Wirkung als vorbekannte Emulgatoren aus dem Stand der Technik auf und eignen sich darüber hinaus für die Verwendung auf einem breiteren Einsatzgebiet als vorbekannte Emulgatoren, wodurch die vorangehende genannte Aufgabe vorteilhaft gelöst wird. Besonders bevorzugt ist ein erfindungsgemäßer Emulgator, wobei der Anteil der Gesamtmenge an Verbindungen der Formel (I) und/oder einem oder mehreren Salz(en) der Verbindung(en) der Formel (I) mit einem oder mehreren durch eine bzw. mehrere Ester-Bindung(en) an das Glycerol-Rückgrat gebundenen Fettsäurerest(en) (ii), bei denen der bzw. einer, mehrere oder sämtliche Fettsäurerest(e) (ii) unabhängig voneinander ausgewählt ist bzw. sind aus der Gruppe, bestehend aus dem Fettsäurerest der Capronsäure, dem Fettsäurerest der Caprylsäure, dem Fettsäurerest der Caprinsäure, dem Fettsäurerest der Laurinsäure und dem Fettsäurerest der Myristinsäure, höher als 70 Gew.-%oder höher als 75 Gew.-%, besonders bevorzugt höher als 90 Gew.-% ist, bezogen auf das Gesamtgewicht der Verbindungen der Formel (I), und/oder
der Anteil der Gesamtmenge an Verbindungen der Formel (I) und/oder einem oder mehreren Salz(en) der Verbindung(en) der Formel (I) mit einem oder mehreren durch eine bzw. mehrere Ester-Bindung(en) an das Glycerol-Rückgrat gebundenen Fettsäurerest (ii), bei denen der bzw. einer, mehrere oder sämtliche Fettsäurerest(e) (ii) unabhängig voneinander ausgewählt ist bzw. sind aus der Gruppe, bestehend aus dem Fettsäurerest der Laurinsäure und dem Fettsäurerest der Myristinsäure, höher als 55 Gew.-%, besonders bevorzugt höher als 60 Gew.-% ist, bezogen auf das Gesamtgewicht der Verbindungen der Formel (I). Überraschenderweise wurde gefunden, dass erfindungsgemäße Emulgatoren mit einem hohen Anteil an durch Ester-Bindungen gebundenen, voranstehend genannten, kurzkettigen Fettsäureresten, insbesondere Fettsäureresten der Capronsäure, Fettsäureresten der Caprylsäure, Fettsäureresten der Caprinsäure, Fettsäureresten der Laurinsäure und Fettsäureresten der Myristinsäure, verbesserte Emulgiereigenschaften aufweisen. Dies äußert sich in verkleinerten Partikelgrößen und einem breiteren Anwendungsgebiet der erfindungsgemäßen Emulgatoren.

Besonders bevorzugt ist ein erfindungsgemäßer Emulgator oder eine Mischung von hierin beschriebenen erfindungsgemäßen Emulgatoren, wobei für eine oder mehrere Verbindungen der Formel (I) gilt, dass der bzw. die Fettsäurerest(e) (ii) unabhängig voneinander ausgewählt ist bzw. sind aus der Gruppe, bestehend aus dem Fettsäurerest der Palmitinsäure, dem Fettsäurerest der Palmitoleinsäure, dem Fettsäurerest der Stearinsäure, dem Fettsäurerest der Ölsäure, dem Fettsäurerest der Linolsäure und dem Fettsäurerest der Linolensäure, niedriger als 35 Gew.-%, besonders bevorzugt niedriger als 35 Gew.-% ist, bezogen auf das Gesamtgewicht der Verbindungen der Formel (I).

Es wurde überraschend festgestellt, dass erfindungsgemäße Emulgatoren mit einem niedrigen Anteil an durch Ester-Bindungen gebundenen, voranstehend genannten, längerkettigen Fettsäureresten, insbesondere dem Fettsäurerest der Palmitinsäure, dem Fettsäurerest der Palmitoleinsäure, dem Fettsäurerest der Stearinsäure, dem Fettsäurerest der Ölsäure, dem Fettsäurerest der Linolsäure und dem Fettsäurerest der Linolensäure, verbesserte Emulgiereigenschaften bereitstellen. Dies äußert sich in verkleinerten Partikelgrößen und einem breiteren Anwendungsgebiet der erfindungsgemäßen Emulgatoren gegenüber vorkannten Emulgatoren.

Im Folgenden werden erfindungsgemäße Verfahren zur Herstellung der hierin beschriebenen erfindungsgemäßen Emulgatoren beschrieben.

Ein weiterer Aspekt der vorliegenden Erfindung betrifft demnach ein Verfahren zur Herstellung eines erfindungsgemäßen Emulgators, umfassend oder bestehend aus den folgenden Schritten:
(A) Umsetzen einer Fettsäurezusammensetzung bestehend aus oder umfassend eine oder mehrere Fettsäuren und/oder einen oder mehrere Fettsäurereste, wobei die bzw. eine, mehrere oder sämtliche Fettsäure(n) ausgewählt ist bzw. sind aus der Gruppe, bestehend aus Capronsäure, Caprylsäure, Caprinsäure, Laurinsäure, Myristinsäure, Palmitinsäure, Palmitoleinsäure, Stearinsäure, Ölsäure, Linolsäure und Linolensäure und/oder einem oder mehreren Fettsäurerest(en), wobei der bzw. ein, mehrere oder sämtliche Fettsäurerest(e) (ii) ausgewählt ist bzw. sind aus der Gruppe, bestehend aus oder umfassend wenigstens Laurinsäure und/oder Myristinsäure und/oder dem Fettsäurerest der Laurinsäure und dem Fettsäurerest der Myristinsäure, wobei die Fettsäurezusammensetzung ausgewählt ist aus der Gruppe bestehend aus einem Öl, einem Fett, einem fetten Öl oder Mischungen daraus oder umfassend solche, mit Glycerol und einer oder mehreren katalytischen Basen,
(B) Zugeben von Citronensäure, einem oder mehreren Salz(en) der Citronensäure oder einer Mischung aus Citronensäure und einem oder mehreren Salz(en) der Citronensäure, zu der in Schritt (A) erhaltenen Mischung, und
(C) optional: Fällen der in Schritt (B) erhaltenen Mischung mit einem oder mehreren Lösungsmitteln zur Anreicherung der Verbindung(en) der Formel (I), und
(D) optional: Aufreinigen der aus Schritt (B) oder (C) erhaltenen Mischung unter Verwendung eines Lösungsmittels oder mehrerer Lösungsmittel ausgewählt aus der

Gruppe, bestehend aus halogenierten, polaren, protisch polaren, protisch unpolaren, "grünen" Lösungsmitteln und Mischungen daraus oder umfassend solche, und/oder Durchführen eines Aufreinigungsverfahrens oder mehrerer Aufreinigungsverfahren ausgewählt aus der Gruppe, bestehend aus Filtration, Ultra- oder Nanofiltration, Ad- und Absorbtionstechniken, Extraktion, Umkristallisation und chromatographischen Methoden und Kombinationen daraus, wobei in der in Schritt (A) eingesetzten Fettsäurezusammensetzung der Anteil der Gesamtmenge an Fettsäuren aus der Gruppe bestehend aus Capronsäure, Caprylsäure, Caprinsäure, Laurinsäure und Myristinsäure und/oder Fettsäureresten aus der Gruppe bestehend aus dem Fettsäurereste der Capronsäure, dem Fettsäurereste der Caprylsäure, dem Fettsäurereste der Caprinsäure, dem Fettsäurereste der Laurinsäure und dem Fettsäurereste der Myristinsäure, höher als 45 Gew.-%, vorzugsweise höher als 55 Gew.-%, ist, bezogen auf das Gesamtgewicht der Fettsäurezusammensetzung, wobei in der in Schritt (A) eingesetzten Fettsäurezusammensetzung der Anteil der Gesamtmenge an
- Laurinsäure und/oder des Fettsäurerests der Laurinsäure höher als 20 Gew.-%, vorzugsweise höher als 35 Gew.-%, besonders bevorzugt höher als 40 Gew.-% ist, vorzugsweise, bezogen auf das Gesamtgewicht der Fettsäurezusammensetzung, und/oder
- Myristinsäure und/oder des Fettsäurerests der Myristinsäure höher als 5 Gew.-%, vorzugsweise höher als 10 Gew.-%, besonders bevorzugt höher als 15 Gew.-% ist, bezogen auf das Gesamtgewicht der Fettsäurezusammensetzung.

Das voranstehend beschriebene Verfahren ist geeignet zur Herstellung erfindungsgemäßer Emulgatoren, wobei je nachdem ob und bis zu welchem Grad die genannten Reinigungsschritte durchgeführt werden, ein erfindungsgemäßer Emulgator bzw. Mischungen erfindungsgemäßer Emulgatoren erhalten werden, auf die im Folgenden, insbesondere in den Beispielen, weiter eingegangen wird. Das voranstehend beschriebene Verfahren ist demnach geeignet, die erfindungsgemäßen Emulgatoren, die geeignet sind, die technische Aufgabe zu lösen, bereitzustellen.

"Umsetzen" im Sinne der vorliegenden Erfindung bedeutet das in Kontakt bringen der jeweiligen Reagenzien in einem zur chemischen Reaktion geeigneten Behältnis oder einer Mehrzahl solcher Behältnisse, bevorzugt unter kontrollierten Bedingungen, umfassend Druck und Temperatur, mit dem Ziel, ein Zwischenprodukt oder Produkt zu erhalten, das also entweder direkt oder nach Aufreinigung verwendet werden kann oder in weiteren Umsetzungen Verwendung findet.

Eine "Fettsäurezusammensetzung" im Sinne der vorliegenden Erfindung ist eine Verbindung oder eine Mischung aus Verbindungen, die geeignet ist, entweder die voranstehend genannten Fettsäurereste und/oder die entsprechenden Fettsäuren bereitzustellen. Erfindungsgemäß umfasst eine Fettsäurezusammensetzung demnach ein oder mehrere Öl(e), ein oder mehrere Fett(e), ein fettes Öl oder mehrere fette Öle, eine oder mehrere Fettsäure(n), für deren Struktur (ii-x) gilt: 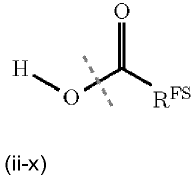 oder Mischungen daraus, wobei der Teil rechts der gestrichelten Linie von (ii-x) erfindungsgemäß vorzugsweise eines der Strukturelemente der Teile rechts der gestrichelten Linie der Verbindungen (ii-a) bis (ii-k) bedeutet. Bezüglich R^{FS} gilt überdies das vorausgehend Gesagte.

"Fällen" im Sinne der vorliegenden Erfindung bezeichnet eine Methode, einen gelösten Stoff durch Zusätze geeigneter Substanzen (Fällungsmittel) ganz oder teilweise als unlöslichen Niederschlag in Form von Kristallen, Flocken oder Tröpfchen auszuscheiden. Dabei ist es gleichgültig, ob durch das Fällungsmittel seine chemische Zusammensetzung verändert wird oder nicht. Die entstehenden Niederschläge von ausgefällten Feststoffen sind zunächst meist mikrokristallin oder amorph. Bei weiterem Kontakt mit der überstehenden Lösung (Mutterlauge) findet im Lauf der Zeit durch Umkristallisation oft eine Vergrößerung der Teilchen und gegebenenfalls eine Umwandlung in stabilere Kristall-Modifikationen statt.

"Aufreinigen" im Sinne der vorliegenden Erfindung bezeichnet alle Verfahren, einzeln oder in ihrer Gesamtheit, die zur Gewinnung der erfindungsgemäßen Erzeugnisse eines bestimmten Reinheitsgrades durch Trennen von den Verunreinigungen, d.h. anderen als den erfindungsgemäßen Erzeugnissen oder sonstigen erwünschten Verbindungen, dienen. Aufreinigungsverfahren umfassen beispielsweise Lösen und (Aus-)Fällen, die Verwendung von Adsorptionsverfahren und Chromatographieverfahren, die Verwendung von Elektrophorese, Schmelzen, Ausfrieren, normales Erstarren, Kristallisieren, Sublimation, Aufwachsverfahren und andere Transportreaktionen, (fraktionierte) Destillation, Rektifikation und viele andere Trennverfahren.

Eine weitere Ausführungsform der vorliegenden Erfindung betrifft demnach ein Verfahren zur Herstellung eines erfindungsgemäßen Emulgators, umfassend oder bestehend aus den folgenden Schritten:
(A) Umsetzen einer Fettsäurezusammensetzung bestehend aus oder umfassend eine oder mehrere Fettsäuren und/oder einen oder mehrere Fettsäurereste, wobei die bzw. eine, mehrere oder sämtliche Fettsäure(n) ausgewählt ist bzw. sind aus der Gruppe, bestehend aus Capronsäure, Caprylsäure, Caprinsäure, Laurinsäure, Myristinsäure, Palmitinsäure, Palmitoleinsäure, Stearinsäure, Ölsäure, Linolsäure und Linolensäure und/oder einem oder mehreren Fettsäurerest(en), wobei der bzw. ein, mehrere oder sämtliche Fettsäurerest(e) (ii) ausgewählt ist bzw. sind aus der Gruppe, bestehend aus oder umfassend wenigstens Laurinsäure und Myristinsäure und/oder dem Fettsäurerest der Laurinsäure und/oder dem Fettsäurerest der Myristinsäure, wobei die Fettsäurezusammensetzung ausgewählt ist aus der Gruppe bestehend aus einem Öl, einem Fett, einem fetten Öl oder Mischungen daraus oder umfassend solche, mit Glycerol und einer oder mehreren katalytischen Basen,
(B) Zugeben von Citronensäure, einem oder mehreren Salz(en) der Citronensäure oder einer Mischung aus Citronensäure und einem oder mehreren Salz(en) der Citronensäure, zu der in Schritt (A) erhaltenen Mischung, und
(C) optional: Fällen der in Schritt (B) erhaltenen Mischung mit einem oder mehreren Lösungsmitteln zur Anreicherung der Verbindung(en) der Formel (I), und
(D) optional: Aufreinigen der aus Schritt (B) oder (C) erhaltenen Mischung unter Verwendung eines Lösungsmittels oder mehrerer Lösungsmittel ausgewählt aus der Gruppe, bestehend aus halogenierten, polaren, protisch polaren, protisch unpolaren, "grünen" Lösungsmitteln und Mischungen daraus oder umfassend solche, und/oder Durchführen eines Aufreinigungsverfahrens oder mehrerer Aufreinigungsverfahren ausgewählt aus der Gruppe, bestehend aus Filtration, Ultra- oder Nanofiltration, Ad- und Absorbtionstechniken, Extraktion, Umkristallisation und chromatographischen Methoden und Kombinationen daraus, wobei in der in Schritt (A) eingesetzten Fettsäurezusammensetzung der Anteil der Gesamtmenge an Fettsäuren aus der Gruppe bestehend aus Capronsäure, Caprylsäure, Caprinsäure, Laurinsäure und Myristinsäure und/oder Fettsäureresten aus der Gruppe bestehend aus dem Fettsäurerest der Capronsäure, dem Fettsäurerest der Caprylsäure, dem Fettsäurerest der Caprinsäure, dem Fettsäurerest der Laurinsäure und dem Fettsäurerest der Myristinsäure, höher als 45 Gew.-%, vorzugsweise höher als 55 Gew.-% ist, bezogen auf das Gesamtgewicht der Fettsäurezusammensetzung, wobei in der in Schritt (A) eingesetzten Fettsäurezusammensetzung der Anteil der Gesamtmenge an Laurinsäure und/oder des Fettsäurerests der Laurinsäure höher als 20 Gew.-%, vorzugsweise höher als 35 Gew.-%, besonders bevorzugt höher als 40 Gew.-% ist, bezogen auf das Gesamtgewicht der Fettsäurezusammensetzung, und/oder Myristinsäure und/oder des Fettsäurerests der Myristinsäure höher als 5 Gew.-%, vorzugsweise höher als 10 Gew.-%, besonders bevorzugt höher als 15 Gew.-% ist, bezogen auf das Gesamtgewicht der Fettsäurezusammensetzung.

Überraschenderweise wurde gefunden, dass das voranstehend beschriebene Verfahren zu vorteilhaften, erfindungsgemäßen Emulgatoren mit einem hohen Anteil an durch Ester-Bindungen gebundenen, hierin genannten, kurzkettigen Fettsäureresten, insbesondere Fettsäureresten der Capronsäure, Fettsäureresten der Caprylsäure, Fettsäureresten der Caprinsäure, Fettsäureresten der Laurinsäure und Fettsäureresten der Myristinsäure, verbesserte Emulgiereigenschaften führt (s. hierzu oben).

Für die Zwecke der Bestimmung des Anteils der Gesamtmenge an Fettsäuren an der Fettsäurezusammensetzung sind vorzugsweise sowohl gebundene Fettsäurereste als auch frei vorliegende Fettsäure der Gesamtmenge der jeweiligen Fettsäure zuzuordnen. Die Bestimmung der jeweiligen Anteile erfolgt wie voranstehend beschrieben vorzugsweise chromatographisch oder gravimetrisch. Beispielsweise kann die hierin beschriebene chromatographische Methode unter Verwendung folgender Geräte, (Verbrauchs- )Materialien und Parameter durchgerührt werden: LC-Gerät: Waters Acquity UPLC, MS-Gerät: Bruker micrOTOF Q-II, Säule: Kinetex RP-C18, 1,7 µm (100 × 2,1 mm), Mobile Phase A: Wasser + 0,1 Gew.-% Ameisensäure, Mobile Phase B: Acetonitril + 0,09 Gew.-% Ameisensäure, Fluss: 0.55 mL/min, Temperatur: 50°C.

Die Obergrenze des in Schritt (A) eingesetzten Anteils der Gesamtmenge an Fettsäuren aus der Gruppe bestehend aus Capronsäure, Caprylsäure, Caprinsäure, Laurinsäure und Myristinsäure und/oder Fettsäureresten aus der Gruppe bestehend aus dem Fettsäurerest der Capronsäure, dem Fettsäurerest der Caprylsäure, dem Fettsäurerest der Caprinsäure, dem Fettsäurerest der Laurinsäure und dem Fettsäurerest der Myristinsäure, beträgt vorzugsweise höchstens 90 Gew.-%, besonders bevorzugt höchstens 85 Gew.-%, bezogen auf das Gesamtgewicht der Fettsäurezusammensetzung. Diese Werte ergeben sich aus der Zusammensetzung, vorzugsweise aus der natürlichen Zusammensetzung, der erfindungsgemäß verwendeten Fettsäurezusammensetzung.

Besonders bevorzugt im Sinne der vorliegenden Erfindung ist eines der vorausgehend beschriebenen erfindungsgemäßen Verfahren, wobei in Schritt (A) Glycerol zum Umsetzen in einer Menge von 0,5 bis 5 mol-Äquivalenten, vorzugsweise in einer Menge von 1,0 bis 2,0 mol-Äquivalenten, besonders bevorzugt in einer Menge von 1,25 bis 1,75 mol-Äquivalenten eingesetzt wird, bezogen auf die gesamte Stoffmenge Fettsäurezusammensetzung.

Es hat sich überraschenderweise gezeigt, dass sich der Einsatz der genannten Reagenzien im vorausgehend genannten Konzentrationsbereich vorteilhaft auf die Herstellung erfindungsgemäßer Emulgatoren auswirkt.

Vorteilhaft im Sinne der vorliegenden Erfindung ist eines der vorausgehend beschriebenen erfindungsgemäßen Verfahren, wobei die bzw. eine, mehrere oder sämtliche katalytische Base(n) aus Schritt (A) in einer Menge von 0,01 bis 0,5 mol-Äquivalenten, vorzugsweise in einer Menge von 0,025 bis 0,1 mol-Äquivalenten, besonders bevorzugt in einer Menge von 0,05 bis 0,1 mol-Äquivalenten eingesetzt wird bzw. werden, bezogen auf die gesamte Stoffmenge der Fettsäurezusammensetzung und/oder wobei die bzw. eine, mehrere oder sämtliche katalytische Base(n) aus Schritt (A) unabhängig voneinander ausgewählt ist bzw. sind aus der Gruppe, bestehend aus Aminbasen, Aminosäuren, Bronsted Basen, Lewis Basen, Alkalisalzen, Erdalkalisalzen und Mischungen daraus, vorzugsweise aus der Gruppe, bestehend aus Natriumhydroxid, Kaliumhydroxid und Mischungen daraus.

Überraschenderweise hat sich herausgestellt, dass der Einsatz der genannten Reagenzien oder im hierin genannten Konzentrationsbereich vorteilhaft auf die Herstellung erfindungsgemäßer Emulgatoren auswirkt. Als besonders bevorzugt hat sich der Einsatz einer oder mehrerer der genannten Reagenzien im hierin genannten Konzentrationsbereich herausgestellt.

Besonders bevorzugt im Sinne der vorliegenden Erfindung ist eines der hierin beschriebenen erfindungsgemäßen Verfahren, wobei Schritt (A) bei einer Temperatur im Bereich von 150 bis 300°C, vorzugsweise im Bereich von 175 bis 275°C, besonders bevorzugt im Bereich von 200 bis 250°C, und optional unter Vakuum-Bedingungen durchgeführt wird.

Eine "Temperatur im Bereich" von einer Untergrenze bis zu einer Obergrenze im Sinne der vorliegenden Erfindung bedeutet, dass sich die Temperatur während der gesamten Reaktionsdauer oder während des wesentlichen Teils der Reaktionsdauer innerhalb des spezifizierten Bereichs befindet. Darunter sind sowohl isotherme Reaktionsbedingungen als auch Reaktionsbedingungen mit Temperaturprofilen zu verstehen, bei denen sich die Temperatur im Wesentlichen innerhalb der spezifizierten Grenzen bewegt. Ein kurzzeitiges Verlassen des Temperaturbereichs für einen nicht wesentlichen Zeitraum steht dem nicht gegenüber.

Selbiges gilt analog für einen "Druck im Bereich" von einer Untergrenze bis zu einer Obergrenze.

"Vakuum" im Sinne der vorliegenden Erfindung bedeutet das Vorliegen eines Drucks in einem Bereich von 900 mbar oder weniger, vorzugsweise in einem Bereich von 100 bis 700 mbar, besonders bevorzugt in einem Bereich von 200 bis 500 mbar.

Wenn nicht explizit anders erwähnt, wird das Verfahren bzw. werden die einzelnen, mehrere oder sämtliche Verfahrensschritte unabhängig voneinander bei Standardbedingungen, also Standardtemperatur und/oder Standardruck durchgeführt. Sofern nur Werte für einen der Parameter angegeben sind, gelten in diesem Fall für den anderen Parameter Standardbedingungen.

Überraschenderweise hat sich herausgestellt, dass das Durchführen von Schritt (A) bei einer Temperatur im vorausstehend genannten Bereich sich vorteilhaft auf die Herstellung erfindungsgemäßer Emulgatoren auswirkt. Weiterhin zeichnen sich erfindungsgemäße Emulgator-Vorprodukte durch ein verbessertes Farb- und Geruchsprofil aus. Dieses verbesserte Farb- und Geruchsprofil ist besonders vorteilhaft für den finalen Farb- und Geruchseindruck der darauf aufbauenden erfindungsgemäßen Emulgatoren bzw. der weiteren erfindungsgemäßen Erzeugnisse. Es wird von den Erfindern angenommen, dass dieses Merkmal in Zusammenhang mit einem geeigneten erfindungsgemäßen Verhältnis an Mono- und Diglyceriden bzw. von Mono- zu Diglyceriden in der zugrundeliegenden Fettsäurezusammensetzung steht, das durch die genannten Verfahrensschritte im Wesentlichen unverändert bleibt.

Besonders bevorzugt im Sinne der vorliegenden Erfindung ist eines der hierin beschriebenen erfindungsgemäßen Verfahren, wobei in Schritt (B) eine Gesamtmenge an Citronensäure und/oder einem oder mehreren Salz(en) davon im Bereich von 0,1 bis 2,0 Gewichtsteilen, vorzugsweise im Bereich von 0,25 bis 1,5 Gewichtsteilen, besonders bevorzugt im Bereich von 0,25 bis 1,0 Gewichtsteilen zugegeben wird, bezogen auf das Gesamtgewicht der in Schritt (A) erhaltenen Mischung.

Der Einsatz von Citronensäure im vorausgehend genannten Konzentrationsbereich hat sich überraschenderweise als vorteilhaft für die Herstellung erfindungsgemäßer Emulgatoren herausgestellt.

Besonders bevorzugt im Sinne der vorliegenden Erfindung ist eines der vorausgehend beschriebenen erfindungsgemäßen Verfahren, wobei für die in Schritt (B) nach erfolgter Zugabe von Citronensäure und/oder einem oder mehreren Salz(en) davon ablaufende Reaktion eine Temperatur im Bereich von 50 bis 200°C, vorzugsweise im Bereich von 100 bis 175°C, besonders bevorzugt im Bereich von 120 bis 160°C und optional Vakuum-Bedingungen eingestellt wird bzw. werden.

Bezüglich der Vakuum-Bedingungen von Schritt (B) gilt selbiges wie für die Vakuum-Bedingungen von Schritt (A). Insbesondere hat sich gezeigt, dass sich die Kombination von Vakuum-Bedingungen sowohl in Schritt (A) als auch in Schritt (B) vorteilhaft auf die Herstellung erfindungsgemäßer Emulgatoren auswirkt.

Überraschenderweise hat sich herausgestellt, dass sich das Durchführen von Schritt (B) bei einer Temperatur im vorausstehend genannten Bereich vorteilhaft auf die Herstellung erfindungsgemäßer Emulgatoren auswirkt. Wie hierin beschrieben, zeichnen sich erfindungsgemäße Emulgatoren vorteilhafterweise durch ein verbessertes Farb- und Geruchsprofil aus.

Vorteilhaft im Sinne der vorliegenden Erfindung ist eines der hierin beschriebenen erfindungsgemäßen Verfahren, wobei das bzw. ein, mehrere oder sämtliche Lösungsmittel in Schritt (C), falls vorhanden, bevorzugt ausgewählt ist bzw. sind aus der Gruppe, bestehend aus Pentan, Hexan, Heptan, Cyclopentan, Cyclohexan, Tetrachlormethan, Diisopropylether, Toluol, Benzol, Diethylether, Dichlormethan, Chloroform, Aceton, Dioxan, Tetrahydrofuran, 2-Methylthetrahydrofuran, tert-Butylmethylether, Ethylacetat, Dimethylsulfoxid, Acetonitril, Benzonitril, Pyridin, 2-Propanol, Ethanol, Methanol, Essigsäure, Ameisensäure, Wasser und Mischungen daraus.

Ein "Lösungsmittel" im Sinne der vorliegenden Erfindung beschreibt einen Stoff, der andere Stoffe auf physikalischem Wege zur Lösung bringen kann, im engeren Sinne eine anorganische oder organische Flüssigkeit, die andere gasförmige, flüssige oder feste Stoffe zu lösen vermag. Als Lösungsmittel oder Lösungsmittelgemisch können auch bevorzugt sein: Wasser, niedere Alkohole (Ethanol, Methanol), Aceton, 1,4-Dioxan, Tetrahydrofuran, tert-Butylmethylether, Propylenglycol, Glycerin, aliphatische Ester aliphatischer Alkohole (wie z.B. Essigsäureethylester, Triacetin), Ether (z.B. Diethylether), Alkane (z.B. Hexan, Heptan), chlorhaltige Lösungsmittel (z.B. Chloroform, Dichlormethan) und aromatische Lösungsmittel (z.B. Benzol, Toluol), pflanzliche Öle oder Fette, oder super- kritisches Kohlendioxid oder Gemische der hierin genannten Lösungsmittel.

Ein "grünes Lösungsmittel" im Sinne der vorliegenden Erfindung erfüllt zusätzlich zu den vorausstehend genannten Bedingungen das Kriterium, die aus der Benutzung des grünen Lösungsmittels resultierende Umweltbelastung zu minimieren. In die Beurteilung der Umweltbelastung fließen neben Gesundheits- und Sicherheitsfragen ebenso energetische Betrachtungen, beispielsweise hinsichtlich des energetischen Aufwands zum Erreichen des Siedepunktes, mit ein (C. Capello et al., What is a green solvent? A comprehensive framework for the environmental assessment of solvents, Green Chemistry, 2007, 9, 927-934).

Besonders bevorzugt im Sinne der vorliegenden Erfindung ist eines der vorausgehend beschriebenen Verfahren, wobei die Fettsäurezusammensetzung ausgewählt ist aus der Gruppe, bestehend aus Babassu-Öl, Palmkern-Öl, Kokos-Öl, Macaúba-Öl, und Mischungen daraus.

"Babassu-Öl" im Sinne der vorliegenden Erfindung ein Pflanzenfett, das vorzugsweise aus den Samen der Babassu- oder Cusipalme (*Attalea speciosa*) gewonnen wird. Erfindungsgemäß kann es in halbfester, talgartiger Form, als flüssiges, klares bis gelbliches Öl oder in raffinierter Form verwendet werden. Mehr bevorzugt ist die Verwendung als hellgelbes Öl. Besonders bevorzugt ist die Verwendung in Form eines raffinierten farblosen, geruchslosen Öls mit Schmelzpunkt nahe Raumtemperatur.

Überraschenderweise hat sich herausgestellt, dass sich der Einsatz der genannten Öle oder Mischungen daraus als erfindungsgemäße Fettsäurezusammensetzungen, vorzugsweise basierend auf natürlichen Rohstoffen, vorteilhaft auf die Herstellung erfindungsgemäßer Emulgatoren auswirkt.

Weitere Öle, die im Sinne der vorliegenden Erfindung verwendet werden können, sind Cohune-(Palm) Öl (*Orbignya cobune*/*Attalea cohune*), Murumuru-Butter (*Astrocaryum murumuru*), Lorbeer-Öl (*laurus nobilis*), Muskatnuss-Öl (*Myristica fragrans*), Afrikanisches Muskatnuss-Öl (*Pycnanthus angolensis*), Pfirsichkernöl (Pejibaye palm, *Bactris gasipaes*)*,* Indaiá-Öl (*Attalea dubia*).

Das hierin beschriebene erfindungsgemäße Verfahren bzw. seine als vorzugsweise oder bevorzugt beschriebenen Ausführungsformen werden erfindungsgemäß genutzt zur Herstellung von erfindungsgemäßen Emulgatoren, Emulgator-Vorprodukten und Mischungen daraus.

Ein weiterer Aspekt der vorliegenden Erfindung betrifft demnach ein Emulgator-Vorprodukt, erhalten durch oder erhältlich durch Aufreinigen der aus Schritt (B) gemäß einem der vorausgehend beschriebenen, erfindungsgemäßen Verfahren erhaltenen Mischung unter Verwendung eines Lösungsmittels oder mehrerer Lösungsmittel ausgewählt aus der Gruppe, bestehend aus halogenierten, polaren, protisch polaren, protisch unpolaren, "grünen" Lösungsmitteln und Mischungen daraus, und/oder durch Durchführen eines Aufreinigungsverfahrens oder mehrerer Aufreinigungsverfahren ausgewählt aus der Gruppe, bestehend aus Filtration, Ultra- oder Nanofiltration, Ad- und Absorbtionstechniken, Extraktion, Umkristallisation und chromatographischen Methoden und Kombinationen daraus.

Das Emulgator-Vorprodukt kann Reste der eingesetzten Edukte sowie des bzw. der katalytischen Base(n) in einer Menge von > 5 % Citronensäure, > 5 % Glycerol und/oder >10 % Triglyceride umfassen.

Bevorzugt ist eine weitere Anreicherung der Verbindungen der Formel (I), die in dem aus den Schritten (B), (C) oder (D) erhaltenen oder erhältlichen erfindungsgemäßen Emulgator enthalten sind, auf Konzentrationen im Bereich von 25 bis 100 Gew.-%, vorzugsweise im Bereich von 45 bis 100 Gew.-%, mehr bevorzugt im Bereich von 75 bis 100 Gew.-% oder im Bereich von 80 bis 100 Gew.-% oder im Bereich von 85 bis 100 Gew.-% oder im Bereich von 90 bis 100 Gew.-%, besonders bevorzugt im Bereich von 95 bis 100 Gew.-% oder im Bereich von 97 bis 100 Gew.-% oder im Bereich von 99 bis 100 Gew.-% durch einen oder mehrere Anreicherungsschritte, bezogen auf das Gesamtgewicht des erfindungsgemäßen Emulgators. Im Sinne der vorliegenden Erfindung ist "Anreicherung" zu verstehen als das Anwenden einer oder mehrerer hierin beschriebener Aufreinigungsverfahren.

Bevorzugt im Sinne der vorliegenden Erfindung ist einer der vorausgehend beschriebenen erfindungsgemäßen Emulgatoren, erhalten durch oder erhältlich durch eines der vorausgehend beschriebenen erfindungsgemäßen Verfahren.

Besonders bevorzugt im Sinne der vorliegenden Erfindung ist einer der vorausgehend beschriebenen erfindungsgemäßen Emulgatoren erhalten durch oder erhältlich durch eines der vorausgehend beschriebenen, wobei das Verfahren Schritt (C) umfasst, und optional wobei der Anteil der Gesamtmenge an Verbindungen der Formel (I) und einem oder mehreren Salz(en) der Verbindung(en) der Formel (I) mit genau einem durch eine Ester-Bindung an das Glycerol-Rückgrat gebundenen Citronensäurerest (iii) höher als 50 Gew.-%, vorzugsweise höher als 85 Gew.-%, ist, bezogen auf das Gesamtgewicht des Emulgators, und vorzugsweise wobei das Verhältnis der Anteile der Gesamtmenge an Verbindungen der Formel (I) und einem oder mehreren Salz(en) Verbindung(en) der Formel (I) mit genau einer durch einen durch eine Ester-Bindung an das Glycerol-Rückgrat gebundenen Fettsäurerest zur Gesamtmenge an Verbindungen der Formel (I) und einem oder mehreren Salz(en) Verbindung(en) der Formel (I) mit genau zwei durch Ester-Bindungen an das Glycerol-Rückgrat gebundenen Fettsäurereste im Bereich von 1:1,5 bis 1:3, vorzugsweise im Bereich von 1:1,75 bis 1:2, besonders bevorzugt im Bereich von 1:2 bis 1:2,5 liegt, bezogen auf das Gewicht dieser Verbindungen.

Es hat sich überraschend gezeigt, dass Emulgatoren, bei denen der Anteil der Gesamtmenge an Verbindungen der Formel (I) und einem oder mehreren Salz(en) der Verbindung(en) der Formel (I) mit genau einem durch eine Ester-Bindung an das Glycerol-Rückgrat gebundenen Citronensäurerest (iii) höher als 50 Gew.-%, vorzugsweise höher als 85 Gew.-%, ist, bezogen auf das Gesamtgewicht des Emulgators, besonders vorteilhafte Eigenschaften aufweisen.

Die besondere Eignung zur Herstellung von Emulsionen verkleinerter Partikelgröße steht nach eigenen Untersuchungen im Zusammenhang mit dem Verhältnis der Anteile der Gesamtmenge an Verbindungen der Formel (I) und einem oder mehreren Salz(en) Verbindung(en) der Formel (I) mit genau einer durch eine Ester-Bindung an das Glycerol-Rückgrat gebundenen Fettsäurerest zur Gesamtmenge an Verbindungen der Formel (I) und einem oder mehreren Salz(en) der Verbindung(en) der Formel (I) mit genau zwei durch Ester-Bindungen an das Glycerol-Rückgrat gebundenen Fettsäurereste im Bereich von 1:1,5 bis 1:3, vorzugsweise im Bereich von 1:1,75 bis 1:2, besonders bevorzugt im Bereich von 1:2 bis 1:2,5 liegt, bezogen auf das Gewicht dieser Verbindungen.

Ein weiterer Aspekt der vorliegenden Erfindung betrifft die Verwendung einer Mischung aus Verbindungen der Formel (I) wie oben definiert als Emulgator.

Eine weitere Ausführungsform der vorliegenden Erfindung betrifft die Verwendung einer Mischung gemäss Anspruch 1 als Emulgator.

Im Folgenden werden Details zu erfindungsgemäßen Emulsionen, erfindungsgemäßen Verfahren zur Herstellung solcher Emulsionen und erfindungsgemäßen Zubereitungen umfassend die vorausgehend beschriebenen erfindungsgemäßen Emulgator-Vorprodukte, Emulgatoren oder Mischungen daraus beschrieben.

Für die in den erfindungsgemäßen Emulsionen und erfindungsgemäßen Zubereitungen enthaltenen Verbindungen der Formel (I) und/oder Salze der Verbindungen der Formel (I) bzw. Mischungen daraus gelten hierbei die obigen Ausführungen.

Die vorliegende Erfindung betrifft demnach weiterhin eine Emulsion umfassend oder bestehend aus einer Öl-Phase, enthaltend eines oder mehrere der hierin beschriebenen erfindungsgemäßen Emulgator-Vorprodukt(e) und/oder einen oder mehrere der hierin beschriebenen erfindungsgemäßen Emulgator(en) oder eine Mischung daraus, und einer wässrigen Phase.

Die Bestandteile der Öl-Phase der erfindungsgemäßen Emulsionen können vorteilhaft ausgewählt werden aus der Gruppe der Ester aus gesättigten und/oder ungesättigten, verzweigten und/oder unverzweigten Alkancarbonsäuren einer Kettenlänge von 3 bis 30 C-Atomen und/oder ihren Salzen und gesättigten und/oder ungesättigten, verzweigten und/oder unverzweigten Alkoholen einer Kettenlänge von 3 bis 30 C-Atomen und/oder ihren Salzen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen und/oder ihren Salzen. Vorzugsweise umfasst die Öl-Phase Bestandteile ausgewählt aus der Gruppe bestehend aus Cetylstearyl Alkohol, Pentaerythrityl Distearat, Cetearyl Octanoat, Persea Gratissima (Avocado) Öl, Capryl- bzw. Caprintriglcerid und Dimethicon. Besonders bevorzugt umfasst die Öl-Phase Bestandteile ausgewählt aus der Gruppe bestehend aus Cetylstearyl Alkohol in einem Anteil von 0,0001 Gew.% bis 50 Gew.-%, vorzugsweise in einem Anteil von 0,01 Gew.% bis 5 Gew.-%, besonders bevorzugt in einem Anteil von 0,5 Gew.% bis 1,5 Gew.-%, Pentaerythrityl Distearat in einem Anteil von 0,0001 Gew.% bis 50 Gew.-%, vorzugsweise in einem Anteil von 0,01 Gew.% bis 5 Gew.-%, besonders bevorzugt in einem Anteil von 0,7 Gew.% bis 1,7 Gew.-%, Cetearyl Octanoat in einem Anteil von 0,0001 Gew.% bis 50 Gew.-%, vorzugsweise in einem Anteil von 1 Gew.% bis 10 Gew.-%, besonders bevorzugt in einem Anteil von 3,5 Gew.% bis 4,5 Gew.-%, Persea Gratissima (Avocado) Öl in einem Anteil von 0,0001 Gew.% bis 50 Gew.-%, vorzugsweise in einem Anteil von 0,01 Gew.% bis 6 Gew.-%, besonders bevorzugt in einem Anteil von 1,5 Gew.% bis 2,5 Gew.-%, Capryl- bzw. Caprintriglcerid in einem Anteil von 0,0001 Gew.% bis 50 Gew.-%, vorzugsweise in einem Anteil von 0,01 Gew.% bis 15 Gew.-%, besonders bevorzugt in einem Anteil von 5 Gew.% bis 7 Gew.-%, und Dimethicon in einem Anteil von 0,0001 Gew.% bis 50 Gew.-%, vorzugsweise in einem Anteil von 0,001 Gew.% bis 3 Gew.-%, besonders bevorzugt in einem Anteil von 0,05 Gew.% bis 1 Gew.-%, bezogen auf das Gesamtgewicht der erfindungsgemäßen Emulsion.

Ferner können Bestandteile der Öl-Phase vorteilhaft ausgewählt werden aus der Gruppe der verzweigten und unverzweigten Kohlenwasserstoffe und -wachse, der Siliconöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnussöl, Rapsöl, Mandelöl, Palmöl, Kokosöl, Palmkernöl und dergleichen mehr.

Die wässrige Phase von Emulsionen im Sinne dieser Erfindung enthält gegebenenfalls vorteilhaft wasserlösliche Pflanzenextrakte, Alkohole, Diole oder Polyole (Niederalkyl), sowie deren Ether, vorzugsweise Ethanol, Isopropanol, Propylenglykol, Glycerin, Ethylenglykol-monoethyl- oder -monobutylether, Propylenglykolmonomethyl, -monoethyl- oder- monobutylether, Diethylenglykolmonomethyl- oder -monoethylether und analoge Produkte, ferner Alkohole (Niederalkyl), z.B. Ethanol, 1,2-Propandiol, Glycerin. Vorzugsweise umfasst die wässrige Phase Bestandteile ausgewählt aus der Gruppe bestehend aus Wasser, demineralisiertem Wasser, Hydroxyacetophenon und Glycerin. Besonders bevorzugt umfasst die wässrige Phase Bestandteile ausgewählt aus der Gruppe bestehend aus Hydroxyacetophenon in einem Anteil von 0,0001 Gew.% bis 50 Gew.-%, vorzugsweise in einem Anteil von 0,01 Gew.% bis 5 Gew.-%, besonders bevorzugt in einem Anteil von 0,2 Gew.% bis 1,2 Gew.-%, und Hydroxyacetophenon in einem Anteil von 0,0001 Gew.% bis 50 Gew.-%, vorzugsweise in einem Anteil von 0,01 Gew.% bis 10 Gew.-%, besonders bevorzugt in einem Anteil von 2,5 Gew.% bis 3,5 Gew.-%, bezogen auf das Gesamtgewicht der erfindungsgemäßen Emulsion.

Es hat sich gezeigt, dass sich die erfindungsgemäßen Emulsionen durch verbesserte Emulgiereigenschaften gegenüber vorbekannten Emulsionen auszeichnen und damit die zugrundeliegende Aufgabe vorteilhaft lösen.

Bevorzugt im Sinne der vorliegenden Erfindung ist eine erfindungsgemäße Emulsion, zudem umfassend eine oder mehrere unterschiedliche Verbindung(en) zur Senkung oder Erhöhung der Viskosität der Emulsion.

Unter Viskosität versteht man die Eigenschaft, insbesondere einer Flüssigkeit, der gegenseitigen laminaren Verschiebung zweier benachbarter Schichten einen Widerstand entgegenzusetzen. Die Viskosität kann demnach auch als Zähigkeit oder innere Reibung verstanden werden. Zur gezielten Einstellung der Viskosität und der Erzielung einer definierten Konsistenz von Emulsionen oder Zubereitungen können diese beispielswese Verdickungsmittel auf organischer (Alginat, Tragant, Xanthan, modifizierte Cellulosen, Carrageenane, etc.) und/oder anorganischer (Bentonit, pyrogene Kieselsäure, Magnesium-Aluminium-Silicate, etc.) Basis enthalten.

Bevorzugt umfasst eine erfindungsgemäße Emulsion ein oder mehrere Verdickungsmittel, welches oder welche vorteilhaft gewählt werden können aus der Gruppe bestehend aus Siliciumdioxid, Aluminiumsilikaten, Polysacchariden bzw. deren Derivate, z.B. Hyaluronsäure, Xanthangummi, Hydroxyropylmethylcellulose, Carbomer (Ultrez-10), jeweils einzeln oder in Kombination. Vorzugsweise umfasst die eine erfindungsgemäße Emulsion Bestandteile ausgewählt aus der Gruppe bestehend aus Xanthangummi, Carbomer (Ultrez-10) und Mischungen davon. Besonders bevorzugt umfasst eine erfindungsgemäße Emulsion Bestandteile ausgewählt aus der Gruppe bestehend aus Xanthangummi in einem Anteil von 0,0001 Gew.% bis 5 Gew.-%, vorzugsweise in einem Anteil von 0,01 Gew.% bis 3 Gew.-%, besonders bevorzugt in einem Anteil von 0,05 Gew.% bis 0,6 Gew.-%, und Carbomer (Ultrez-10) in einem Anteil von 0,0001 Gew.% bis 5 Gew.-%, vorzugsweise in einem Anteil von 0,01 Gew.% bis 3 Gew.-%, besonders bevorzugt in einem Anteil von 0,05 Gew.% bis 0,7 Gew.-%, bezogen auf das Gesamtgewicht der erfindungsgemäßen Emulsion.

Die vorliegende Erfindung betrifft zudem eine Verwendung einer erfindungsgemäßen Emulsion in einer der Reinigung dienenden Zubereitung, einer kosmetischen oder pharmazeutischen, vorzugsweise einer dermatologischen, Zubereitung, einer dem Genuss oder der Ernährung dienenden Zubereitung oder in einer Halbfertigware zur Herstellung einer solchen Zubereitung.

Eine kosmetische oder pharmazeutische, vorzugsweise dermatologische Zubereitung im Sinne der vorliegenden Erfindung ist vorzugsweise eine Zubereitung, die unter anderem bevorzugt der kosmetischen Hautpflege dient. Unter kosmetischer Hautpflege ist in erster Linie zu verstehen, die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) zu stärken oder wiederherzustellen sowie ihre Hornschicht bei auftretenden Schäden in ihrem natürlichen Regenerationsvermögen zu unterstützen. Werden die Barriereeigenschaften der Haut gestört, kann es zu verstärkter Resorption toxischer oder allergener Stoffe oder zum Befall von Mikroorganismen und als Folge zu toxischen oder allergischen Hautreaktionen kommen. Ziel der Hautpflege ist es ferner, den durch tägliches Waschen verursachten Fett-und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Vorzugsweise sind unter pharmazeutischen Zubereitungen im Sinne der Erfindung Zubereitungen zu verstehen, die z.B. in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen vorliegen und als verschreibungspflichtige, apothekenpflichtige oder sonstige Arzneimittel oder als Nahrungsergänzungsmittel verwendet werden.

Medizinische topische Zusammensetzungen, als weitere Beispiele pharmazeutischer Zubereitungen, enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Die kosmetischen und pharmazeutischen Zubereitungen im Sinne dieser Erfindung können Hilfsstoffe enthalten, wie sie üblicherweise in solchen Zubereitungen verwendet werden, z.B. Konservierungsmittel, Antioxidantien, Vitamine, Bakterizide, Parfüme, Substanzen zum Verhindern des Schäumens, Farbstoffe, Pigmente, die eine färbende Wirkung haben, Verdickungsmittel, Tenside, Emollients, Emulgatoren, anfeuchtende und/oder feuchthaltende Substanzen, Feuchtigkeitsvermittlern, Fette, Öle, Wachse, Pflanzenextrakte oder andere übliche Bestandteile wie Alkohole, Niederalklyalkohole, Polyole, Niederalkylpolyole, Polymere, Schaumstabilisatoren, Komplexbildner, Elektrolyte, organische Lösungsmittel, Treibgase, Silikone oder Silikonderivate.

Die erfindungsgemäßen Emulgatoren können vorteilhafterweise in kosmetische und/oder pharmazeutische, insbesondere dermatologische, Zubereitungen eingearbeitet werden, die wie üblich zusammengesetzt sind und dem kosmetischen und/oder dermatologischen Lichtschutz, ferner zur Behandlung, der Pflege und der Reinigung der Haut und/oder der Haare und als Schminkprodukt in der dekorativen Kosmetik dienen. Entsprechend können derartige Zubereitungen, je nach ihrem Aufbau beispielsweise als Hautschutzcreme, Reinigungsmilch, Sonnenschutzlotion, Nährcreme, Tages- oder Nachtcreme usw. verwendet werden. So können derartige Zubereitungen beispielsweise als Emulsion, Lotion, Milch, Creme, Hydrodispersionsgel, Balm, Spray, Schaum, Haar-Shampoo, HaarPflegemittel, Haar-Conditioner, Roll-on, Stick oder Make-up vorliegen.

Der Ernährung oder dem Genuss dienende Zubereitungen im Sinne der vorliegenden Erfindung sind z.B. Backwaren (z.B. Brot, Trockenkekse, Kuchen, sonstiges Gebäck), Süßwaren (z.B. Schokoladen, Schokoladenriegelprodukte, sonstige Riegelprodukte, Fruchtgummi, Hart- und Weichkaramellen, Kaugummi), alkoholische oder nichtalkoholische Getränke (z.B. Kaffee, Tee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke, Instant-Kaffeegetränke, Instant-Fruchtgetränke), Fleischprodukte (z.B. Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (z.B. Frühstückscerealien, Müsliriegel, vorgegarte Fertigreis-Produkte), Milchprodukte (z.B. Milchgetränke, Buttermilchgetränke, Milcheis, Joghurt, Kefir, Frischkäse, Weichkäse, Hartkäse, Trockenmilchpulver, Molke, Butter, Buttermilch, teilweise oder ganz hydrolisierte Milchprotein-haltige Produkte), Produkte aus Sojaprotein oder anderen Sojabohnen-Fraktionen (z.B. Sojamilch und daraus gefertigte Produkte, Fruchtgetränke mit Sojaprotein, Sojalecithin-haltige Zubereitungen, fermentierte Produkte wie Tofu oder Tempe oder daraus gefertigte Produkte), Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegarte Gemüse, eingekochte Gemüse), Knabberartikel (z.B. gebackene oder frittierte Kartoffelchips oder Kartoffelteigprodukte, Extrudate auf Mais- oder Erdnussbasis), Produkte auf Fett-und Ölbasis oder Emulsionen derselben (z.B. Mayonnaise, Remoulade, Dressings), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden. Die Zubereitungen im Sinne der Erfindung können auch als Halbfertigware zur Herstellung weiterer der Ernährung oder dem Genuss dienenden Zubereitungen dienen. Die Zubereitungen im Sinne der Erfindung können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen als Nahrungsergänzungsmittel vorliegen.

Bevorzugt können die erfindungsgemäßen Zubereitungen eine Aromakomposition enthalten, um den Geschmack und/oder Geruch der Zubereitung abzurunden und zu verfeinern. Geeignete Aromakompositionen enthalten z.B. synthetische, natürliche oder naturidentische Aroma-, Riech- und Geschmacksstoffe. Bevorzugte Aromakompositionen umfassen beispielsweise Vanillin, Ethylvanillin, Ethylvanillinisobutyrat (3-Ethoxy-4-isobutyryloxybenzaldehyd), Furaneol (2,5-Dimethyl-4-hydroxy-3(2H)-furanon) und Abkömmlinge (z.B. Homofuraneol, 2-Ethyl-4-hydroxy-5-methyl-3(2H)-furanon), Homofuronol (2-Ethyl-5-methyl-4-hydroxy-3(2H)-furanon und 5-Ethyl-2-methyl-4-hydroxy-3(2H)-furanon), Maltol und Abkömmlinge (z.B. Ethylmaltol), Cumarin und Abkömmlinge, gamma-Lactone (z.B. gamma-Undecalacton, gamma-Nonalacton), delta-Lactone (z.B. 4-Methyldeltalacton, Massoilacton, Deltadecalacton, Tuberolacton), Methylsorbat, Diacetyl, 4-Hydroxy-2(oder 5)-ethyl-5(oder 2)-methyl-3(2H)furanon, 2-Hydroxy-3-methyl-2-cyclopentenone, 3-Hydroxy-4,5-dimethyl-2(5H)-furanon, Fruchtester und Fruchtlactone (z.B. Essigsäure-n-butylester, Essigsäureisoamylester, Propionsäureethylester, Buttersäureethylester, Buttersäure-n-butylester, Buttersäureisoamylester, 3-Methylbuttersäureethylester, *n*-Hexansäureethylester, *n*-Hexansäureallylester, *n-*Hexansäure-*n*-butylester, *n*-Octansäureethylester, Ethyl-3-methyl-3-phenylglycidat, Ethyl-2-trans-4-cis-decadienoat), 4-(p-Hydroxyphenyl)-2-butanon, 1,1-Dimethoxy-2,2,5-trimethyl-4-hexan, 2,6-Dimethyl-5-hepten-1-al und Phenylacetaldehyd.

Im Lichte der obigen Ausführungen wird klar, dass die vorliegende Erfindung auch eine der Reinigung dienende Zubereitung, kosmetische oder pharmazeutische, vorzugsweise dermatologische, Zubereitung, dem Genuss oder der Ernährung dienende Zubereitung, vorzugsweise eine Zubereitung ausgewählt aus den oben beschriebenen Gruppen, oder Halbfertigware, umfassend ein erfindungsgemäßes oder mehrere erfindungsgemäße Emulgator-Vorprodukt(e) oder einen oder mehrere erfindungsgemäße Emulgator(en) oder eine oder mehrere erfindungsgemäße Emulsion(en), betrifft.

Eine der Reinigung dienende Zubereitung im Sinne der vorliegenden Erfindung ist vorzugsweise eine Zubereitung zur Reinigung von Oberflächen (unter anderem Glas, Keramik, Granit, Metall (insbesondere Edelstahl), unbehandeltes Holz, behandeltes Holz) im Haushalts- und/oder Industriebereich.

Die erfindungsgemäßen, der Ernährung oder dem Genuss dienenden Zubereitungen sind regelmäßig Produkte, die dazu bestimmt sind, in die menschliche Mundhöhle eingebracht zu werden, dort eine bestimmte Zeit zu verbleiben und anschließend entweder verzehrt (beispielsweise verzehrfertige Lebensmittel) oder wieder aus der Mundhöhle entfernt zu werden. Zu diesen Produkten gehören dabei sämtliche Erzeugnisse oder Stoffe, die dazu bestimmt sind, in verarbeitetem, teilweise verarbeitetem oder unverarbeitetem Zustand vom Menschen aufgenommen zu werden. Oral konsumierbare Zubereitungen können auch Erzeugnisse sein, die Lebensmitteln bei ihrer Herstellung, Verarbeitung oder Bearbeitung zugesetzt werden und dazu vorgesehen sind, in die menschliche Mundhöhle eingebracht zu werden. Demnach können solche Zubereitungen (hier dann als Halbfertigware) wiederum in (weiteren) der Ernährung oder dem Genuss dienenden gebrauchs- oder verzehrfertigen Zubereitungen enthalten sein (der Ernährung oder dem Genuss dienende gebrauchs- oder verzehrfertige Zubereitungen sind im Rahmen des vorliegenden Textes, insbesondere Lebensmittel, speziell verzehrfertige Lebensmittel).

"Halbfertigware" bezeichnet im Sinne der vorliegenden Erfindung nicht vollständig fertiggestellte Erzeugnisse, wie beispielsweise Emulsionen oder Zubereitungen, die zu einem späteren Zeitpunkt zu Fertigerzeugnissen weiterverarbeitet werden. In diesem einen oder mehreren abschließenden Verarbeitungsschritten können der Halbfertigware weitere wesentliche Verbindungen zugesetzt werden, Verdünnungsschritte erfolgen oder die Halbfertigware verschiedenen mechanischen Prozessen unterworfen werden, um bestimmte makroskopische Eigenschaften zu erreichen. Halbfertigware bezieht sich hierbei auf Lebensmittel, die dazu bestimmt sind, erst im weiter verarbeiteten Zustand, z.B. nach Hinzufügen von für den sensorischen Eindruck (mit)entscheidenden Aroma- oder Geschmacksstoffen verzehrt zu werden.

Ein weiterer Aspekt der vorliegenden Erfindung betrifft ein Verfahren zur Herstellung einer erfindungsgemäßen Emulsion, umfassend oder bestehend aus die folgenden Schritte:
- Bereitstellen einer Öl-Phase, enthaltend ein erfindungsgemäßes Emulgator-Vorprodukt oder einen erfindungsgemäßen Emulgator oder eine Mischung daraus, und einer wässrigen Phase,
- optional: getrenntes Erwärmen der Öl-Phase und der wässrigen Phase, vorzugsweise auf eine Temperatur im Bereich von 40 bis 100°C, vorzugsweise im Bereich von 60 bis 85°C,
- optional: Zugeben einer oder mehrerer unterschiedlicher Verbindungen zur Senkung oder Erhöhung der Viskosität zur erwärmten Öl-Phase,
- Mischen der optional erwärmten wässrigen Phase und der optionale erwärmten Öl-Phase, und
- optional: Abkühlen unter Rühren.

"Bereitstellen" zum Zwecke der Herstellung der erfindungsgemäßen Emulsionen im Sinne der vorliegenden Erfindung bedeutet das Vorlegen der jeweiligen Phasen in einem geeigneten Behältnis oder einer Mehrzahl solcher Behältnisse, bevorzugt unter kontrollierten Bedingungen, umfassend Druck und Temperatur, bevorzugt mit dem Ziel, die nachfolgenden Verfahrensschritte zur Herstellung erfindungsgemäßer Emulsionen zu ermöglichen.

Bei manchen erfindungsgemäßen Öl-Phasen bzw. wässrigen Phasen ist es vorteilhaft oder erforderlich, die jeweils bereitgestellten Phasen zu erwärmen, um die Herstellung einer erfindungsgemäßen Emulsion zu gewährleisten. Der Schritt des Erwärmens dient in der Regel dazu, die jeweiligen Phase zu homogenisieren bzw. zu deren Homogenisierung beizutragen. Dieser Schritt kann beispielsweise durch Rühren, oder sonstige dem Fachmann aus dem Stand der Technik bekannte Maßnahmen zur Förderung der Konvektion zusätzlich gefördert bzw. unterstützt werden.

"Mischen" im Sinne der vorliegenden Erfindung beschreibt das Vereinigen von Verbindungen, sodass eine möglichst gleichmäßige Mischung, also Homogenität, erreicht wird. Für das Vermischen ist es notwendig, dass eine Relativbewegung partieller Volumen-Elemente der zu vermischenden Verbindungen zueinander vorhanden ist. Diese kann aufgrund von Diffusion, Konvektion oder durch von außen eingetragene, mechanische Energie zustande kommen. Mischen dient unter anderem der Herstellung von Lösungen, Emulsionen und/oder dem Wärmetransport. Beim Mischen treten nicht selten Temperaturänderungen im System ein, insbesondere beim Mischen von Flüssigkeiten, ohne dass chemische Reaktionen stattfinden (aufgrund der Mischungsenthalpie). Außerdem kann es zu Volumen-Kontraktionen bzw. -Zunahmen kommen. Das Mischen erfolgt unter anderem durch Rühren, Emulgieren, Lösen, Ultraschall-Einwirkung in Abhängigkeit vom Aggregatzustand und den Eigenschaften der zu mischenden Komponenten. Bei den Mischgeräten (Mischern) kann man prinzipiell zwischen statischen und dynamischen Mischern unterscheiden. Während erstere durch Turbulenz wirken, die an speziell geformten Konstruktionen in Flüssigkeiten oder Gasen beim Durchströmen entsteht, wird diese bei den dynamischen Mischern aktiv erzeugt. Mischer-Typen sind unter anderem Propeller-, Turbo-, Schaufel-, Mulden-, Planeten-, Reib-, Schnecken-, Walzen-, Schleuder-, Gegenstrom-, Strahl-, Trommel-, Konus-, Taumel-, Kreisel-, Kühl-, Vakuum-, Durchfluss-, Schwerkraft-, Fluid- und pneumatische Mischer.

"Abkühlen" im Sinne der vorliegenden Erfindung ist das optionale Abkühlen der aus den vorausgehenden Schritten erhaltenen Mischung, sofern diese bei gegenüber Raumtemperatur oder Standardbedingungen erhöhten Temperaturen hergestellt wurde, auf eine niedrigere Temperatur, bevorzugt zurück auf Raumtemperatur oder die Temperatur bei Standardbedingungen. Vorzugsweise erfolgt das Abkühlen unter Verwendung von im Stand der Technik bekannten Kühlern oder Wärmetauschern. Ebenso kann ein Mischen bzw. Rühren das Abkühlen der Mischung entsprechend der Ausführungen des vorhergehenden Absatzes fördern.

Im Folgenden wird die vorliegende Erfindung anhand von ausgewählten Beispielen näher erläutert, wobei die vorliegende Erfindung nicht hierauf beschränkt ist.

Sofern nicht anders angegeben, beziehen sich sämtliche der folgenden (%-) Angaben auf das Gewicht und sind als "Gew.-%" zu verstehen, bezogen auf das Gesamtgewicht der jeweiligen Zusammensetzung bzw. des jeweiligen Emulgators oder sonstigen spezifizierten Bezugsgesamtheit.

### Beispiel 1: Beispielhafte Fettsäurezusammensetzungen (als Ausgangsmaterial).

Tabelle 1 zeigt eine Übersicht über exemplarisch verwendete Öle und deren Zusammensetzung hinsichtlich ihrer Fettsäurereste (FS). Die Angaben vom Typ "Cx:y" in der linken Spalte ("FS") stehen dabei für die Anzahl der Kohlenstoffatome x und die Anzahl der Doppelbindungen y im jeweiligen Fettsäurerest. So bedeutet beispielsweise "C18:1" einen Fettsäurerest mit 18 Kohlenstoffatomen und einer Doppelbindung. In Klammern wird jeweils der Trivialname der zugrundeliegenden Säure angegeben.

**Tabelle 1 Zusammensetzung hinsichtlich der Fettsäurereste der verwendeten Fettsäurezusammensetzungen.**

| **FS** | **Babassu** | **Palmkern** | **Kokos** | **Macaüba** | **Neutral** | **Sonnenblume** |
|---|---|---|---|---|---|---|
| C6:0 (Capronsäure) | 0,3 % | 0,2 % | 0,6 % | 0,18 % | 0,03 % | -/- |
| C8:0 (Caprylsäure) | 5,6 % | 3,2 % | 7,3 % | 5,61 % | 48,3 % | 0,04 % |
| C10:0 (Caprinsäure) | 5,3 % | 3,2 % | 5,9 % | 4,9 % | 33,3 % | 0,03 % |
| C12:0 (Laurinsäure) | 46,5 % | 45,3 % | 47,9 % | 43,5 % | 0,09 % | 0,01 % |
| C14:0 (Myristinsäure) | 17,5 % | 15,6 % | 19,0 % | 10,6 % | -/- | 0,07 % |
| C16:0 (Palmitinsäure) | 9,8 % | 8,7 % | 8,5 % | 8,0 % | -/- | 6,8 % |
| C18:0 (Stearinsäure) | 3,9 % | 2,4 % | 3,2 % | 3,8 % | -/- | 3,7 % |
| C18:1 (Ölsäure) | 14,9 % | 15,3 % | 4,7 % | 19,7 % | -/- | 31,3 % |
| C18:2 (Linolsäure) | 2,4 % | 2,4 % | 0,7 % | 2,3 % | -/- | 61,4 % |

### Beispiel 2: Anteile an Mono-Glycerid Citrat (MGC) und Diglycerid Citrat (DGC) in Vergleichsproben und erfindungsgemäßen Emulgatoren.

Citronensäureester von Mono- und Diglyceriden von Speisefettsäuren sind seit langem bekannt und werden unter anderem mit der Bezeichnung E472c vertrieben. Verbindungen bestehend aus einer Fettsäure bzw. einem Fettsäurerest, einer oder mehrerer Glycerin-Einheiten, die ihrerseits gegebenenfalls über eine oder mehrere Ether-Bindungen verknüpft sind und einer Citronensäure bzw. einem Citronensäurerest werden als Mono-Glycerid Citrate (MGC) bezeichnet. Verbindungen mit zwei Fettsäuren bzw. Fettsäureresten, einer oder mehrerer Glycerin-Einheiten, die ihrerseits gegebenenfalls über eine oder mehrere Ether-Bindungen verknüpft sind und einer Citronensäure bzw. einem Citronensäurerest werden als Diglycerid Citrate (DGC) bezeichnet. Die Ergebnisse der hierin beschriebenen HPLC-MS Analyse erfindungsgemäßer Emulgatoren und weiterer Vergleichsproben (siehe Tabelle 2) zeigen, dass der Anteil an MGC und DGC für den eigentlichen emulgierenden Effekt in kosmetischen Formulierungen verantwortlich sind.

**Tabelle 2 Anteile an MGC und DGC in Vergleichsproben und erfindungsgemäßen Emulgatoren.**

| **Bezeichnung** | **Rohmaterial** | **(Vergleichs-) Probe / Name** | **MGC / [%]** | **DGC / [%]** | **Gesamt / [%]** |
|---|---|---|---|---|---|
| Dracorin CE | Glycerylstearat Citrate | Probe 1 | 7,6 | 2,16 | 10 |
| Dracorin GOC | Glyceryloleat Citrate | Probe 2 | 8,3 | 7,3 | 16 |
| Eco_Emuls Palmk. | Citronensäurester Mono- und Diglycerid, (Palmkernöl-basiert) | Eco Emulsifier 1 | 7,2 | 18,9 | 26 |
| Eco_Emuls Babassu (Labor-Versuch) | Citronensäurester Mono- und Diglycerid (Babassu-Öl basiert) | Eco Emulsifier 2 | 8,3 | 17,7 | 26 |
| Eco_Emuls coco (Labor-Versuch) | Citronensäurester Mono- und Diglycerid (Kokosöl-basiert) | Eco Emulsifier 3 | 9 | 19 | 28 |

Vergleichsweise analysierte Emulgator-Proben, die nicht in die Kategorie der erfindungsgemäßen Emulgatoren fallen, zeigten prozentuale Anteile von MGC und DGC in Bereichen, die sich deutlich von denen der erfindungsgemäßen Emulgatoren unterscheiden.

Typische aus dem Stand der Technik bekannte, auf Glycerylstearat-Citraten basierende Emulgatoren weisen beispielsweise nach eigenen Untersuchungen einen Gehalt an MGC im Bereich von 7,6 bis 20,4 Gew.-%, einen Gehalt an DGC im Bereich von 1,0 bis 2,6 Gew.-% und hinsichtlich der Summe der Anteile von MGC und DGC einen Gehalt im Bereich von 10 bis 15 Gew.-% auf, bezogen auf das Gesamtgewicht des Emulgators.

Typische aus dem Stand der Technik bekannte, auf Citronensäureester-Mono- und Diglyceriden von Palm- und Sonnenblumenöl basierende Emulgatoren weisen beispielsweise nach eigenen Untersuchungen einen Gehalt an MGC im Bereich von 5 bis 7,2 Gew.-%, einen Gehalt an DGC im Bereich von 3,9 bis 14,2 Gew.-% und hinsichtlich der Summe der Anteile von MGC und DGC einen Gehalt im Bereich von 11 bis 19 Gew.-% auf, bezogen auf das Gesamtgewicht des Emulgators.

Typische aus dem Stand der Technik bekannte, auf Glyceryloleat-Citraten basierende Emulgatoren weisen beispielsweise nach eigenen Untersuchungen einen Gehalt an MGC im Bereich von 8,3 bis 11,2 Gew.-%, einen Gehalt an DGC im Bereich von 6,2 bis 7,3 Gew.-% und hinsichtlich der Summe der Anteile von MGC und DGC einen Gehalt im Bereich von 16 bis 17 Gew.-% auf, bezogen auf das Gesamtgewicht des Emulgators.

"Eco Emulsifier" in den hierin gezeigten Tabellen bezeichnen beispielhafte erfindungsgemäße Emulgatoren. Die Fettsäurezusammensetzung des finalen erfindungsgemäßen Emulgators bzw. "Eco Emulsifiers" spiegelt die Fettsäureverteilung der Ausgangsmaterialien wieder. Normiert auf 100 Gew.-%, d.h. bezüglich der Summe der Anteile des Gewichts der jeweiligen Fettsäuren bzw. Fettsäurereste, sind die erfindungsgemäßen "Eco Emulsifier" durch folgende Fettsäurezusammensetzung (Zuordnung der Fettsäuren nach den Ausführungen in Beispiel 1) charakterisiert.

Eco Emulsifier 1: 0,19 Gew.-% C6:0, 3,36 Gew.-% C8:0, 3,35 Gew.-% C10:0, 47,30 Gew.-% C12:0, 16,25 Gew.-% C14:0, 9,07 Gew.-% C16:0, 2,52 Gew.-% C18:0, 15,62 Gew.-% C18:1 und 2,27 Gew.-% C18:2.

Eco Emulsifier 2: 0,27 Gew.-% C6:0, 5,10 Gew.-% C8:0, 4,94 Gew.-% C10:0, 43,8 Gew.-% C12:0, 16,65 Gew.-% C14:0, 9,29 Gew.-% C16:0, 3,68 Gew.-% C18:0, 14,14 Gew.-% C18:1 und 2,17 Gew.-% C18:2.

Eco Emulsifier 3: 0,35 Gew.-% C6:0, 4,97 Gew.-% C8:0, 3,95 Gew.-% C10:0, 38,6 Gew.-% C12:0, 21,36 Gew.-% C14:0, 13,03 Gew.-% C16:0, 2,58 Gew.-% C18:0, 13,00 Gew.-% C18:1 und 3,23 Gew.-% C18:2.

Die hierin exemplarisch gezeigten bzw. untersuchten "Eco Emulsifier" können vorzugsweise hergestellt werden durch die in den nachfolgenden Beispielen 5 und 6 beschriebenen Verfahren, wobei Babassu-Öl durch das jeweilige spezifizierte "Rohmaterial" zu ersetzen ist. So ist beispielsweise zum Erhalten des "Kokos-Öl basierten Eco Emulsifier 3" eines der erfindungsgemäßen Verfahren aus den Beispielen 5 oder 6 unter Verwendung von Kokos-Öl anstelle des darin verwendeten Babassu-Öls durchzuführen.

### Beispiel 3: Methoden zur Bestimmung des Gehalts an MGC und DGC

Wie vorausgehend beschrieben, kann der Gehalt an MGC und DGC der erfindungsgemäßen Emulgatoren und der Vergleichsproben unter anderem vorzugsweise mittels zweier Methoden bestimmt werden.

In der ersten Methode wird zur Auswertung die Kopplung von Flüssigchromatographie (HPLC, engl.: englisch high performance liquid chromatography) mit Massenspektrometrie (MS) verwendet, die sogenannte HPLC-MS. Die Zuordnung der Peaks in den erhaltenen MS-Spektren erfolgt über die Masse und Retentionsindices. Eine Abschätzung der Menge erfolgt über Integration der jeweiligen Peaks (sogenannte Flächen-%).

Die zweite verwendete Methode basiert auf Fällung und gravimetrischer Analyse.

Ein Vergleich der Mengen an MGC und DGC aus den beiden vorausgehend genannten Methoden (siehe Tabelle 3) zeigt, dass die Werte innerhalb üblicher Fehlergrenzen übereinstimmen.

**Tabelle 3 Methodenvergleich HPLC-MS vs. Gravimetrie zur Bestimmung der Anteile an MGC und DGC in (weiteren) erfindungsgemäßen Emulgatoren.**

| **Rohmaterial** | **Probe / Name** | **MGC / [%]** | **DGC / [%]** | **Citrat gesamt / [%]** | **Gravimetrisch / [%]** |
|---|---|---|---|---|---|
| Citronensäure-Ester Mono- und Diglycerid (Babassu-Öl basiert) | Eco Emulsifier 4 | 8,25 | 17,2 | 25,45 | 23 |
| Citronensäure-Ester Mono- und Diglycerid (Palmkernöl basiert) | Eco Emulsifier 5 | 7,18 | 18,9 | 26,08 | 24 |
| Citronensäure-Ester Mono- und Diglycerid (Kokos-Öl basiert, BRA) | Eco Emulsifier 6 | 8,4 | 21,5 | 29,9 | 30 |
| Citronensäure-Ester Mono- und Diglycerid (Kokos-Öl basiert, PHL) | Eco Emulsifier 7 | 8,6 | 20,6 | 29,2 | 26 |
| Mono- und Diglycerid (Kokos-Öl basiert, LKA) | Eco Emulsifier 8 | 12,8 | 19,8 | 32,6 | 27 |

Teilweise wird in der vorausgehenden Tabelle das Ursprungsland der zugrundeliegenden Öle genannt, wobei "BRA" für Brasilien, "PHL" für die Philippinen und "LKA" für Sri Lanka steht. Die Fettsäurezusammensetzung des finalen Emulgators bzw. "Eco Emulsifiers" spiegelt die Fettsäureverteilung der Ausgangsmaterialien wieder. Normiert auf die 100 Gew.-%, d.h. bezüglich der Summe der Anteile des Gewichts der jeweiligen Fettsäuren bzw. Fettsäurereste, sind die erfindungsgemäßen "Eco Emulsifier" durch folgende Fettsäurezusammensetzung (Zuordnung der Fettsäuren nach den Ausführungen in Beispiel 1) charakterisiert.

Eco Emulsifier 4: 0,31 Gew.-% C6:0, 5,09 Gew.-% C8:0, 4,93 Gew.-% C10:0, 44,23 Gew.-% C12:0, 17,22 Gew.-% C14:0, 9,92 Gew.-% C16:0, 3,96 Gew.-% C18:0, 12,87 Gew.-% C18:1, 1,23 Gew.-% C18:2.

Eco Emulsifier 5: 0,19 Gew.-% C6:0, 3,30 Gew.-% C8:0, 3,36 Gew.-% C10:0, 48,71 Gew.-% C12:0, 17,18 Gew.-% C14:0, 9,83 Gew.-% C16:0, 2,78 Gew.-% C18:0, 15,01 Gew.-% C18:1 und 1,96 Gew.-% C18:2.

Eco Emulsifier 6: 0,38 Gew.-% C6:0, 5,21 Gew.-% C8:0, 4,16 Gew.-% C10:0, 40,78 Gew.-% C12:0, 21,72 Gew.-% C14:0, 12,56 Gew.-% C16:0, 2,21 Gew.-% C18:0, 10,4 Gew.-% C18:1 und 2,45 Gew.-% C18:2.

Eco Emulsifier 7: 0,48 Gew.-% C6:0, 7,16 Gew.-% C8:0, 5,99 Gew.-% C10:0, 49,20 Gew.-% C12:0, 19,60 Gew.-% C14:0, 8,89 Gew.-% C16:0, 3,37 Gew.-% C18:0, 4,64 Gew.-% C18:1 und 0,68 Gew.-% C18:2.

Eco Emulsifier 8: 0,51 Gew.-% C6:0, 7,69 Gew.-% C8:0, 5,79 Gew.-% C10:0, 48,90 Gew.-% C12:0, 20,20 Gew.-% C14:0, 8,06 Gew.-% C16:0, 3,32 Gew.-% C18:0, 4,64 Gew.-% C18:1 und 0,68 Gew.-% C18:2.

Die hierin exemplarisch gezeigten bzw. untersuchten "Eco Emulsifier" können vorzugsweise hergestellt werden durch die in den nachfolgenden Beispielen 5 und 6 beschriebenen Verfahren, wobei das jeweilige spezifizierte "Rohmaterial" anstelle von Babassu-Öl zu verwenden ist. So ist beispielsweise zum Erhalten des "Kokos-Öl basierten Eco Emulsifier 6" eines der erfindungsgemäßen Verfahren aus den Beispielen 5 oder 6 unter Verwendung von Kokos-Öl anstelle des darin verwendeten Babassu-Öls durchzuführen.

### Beispiel 4: Relativer Anteil von DGC zu MGC in erfindungsgemäßen Emulgatoren

Erfindungsgemäße Emulgatoren zeichnen sich vorzugsweise durch ein bestimmtes Verhältnis von DGC zu MGC der erfindungsgemäßen Emulgatoren aus, wobei die Summe der Anteile von MGC und DGC auf 100% normiert wurde (vgl. Tabelle 4).

**Tabelle 4 Verhältnis DGC/MGC beispielhafter erfindungsgemäßer Emulgatoren, wobei die Summe der Anteile von MGC und DGC auf 100% normiert wurde.**

| **Rohmaterial** | **Probe / Name** | **MGC / [%]** normiert | **DGC / [%]** normiert | **Verhältnis DGC** / **MGC** |
|---|---|---|---|---|
| Citronensäure-Ester Mono- und Diglycerid (Babassu-Öl basiert) | Eco Emulsifier 4 | 29,5 | 70,5 | 2,39 |
| Citronensäure-Ester Mono- und Diglycerid (Palmkern-Öl basiert) | Eco Emulsifier 5 | 30,1 | 69,9 | 2,32 |
| Citronensäure-Ester Mono- und Diglycerid (Kokos-Öl basiert, BRA) | Eco Emulsifier 6 | 28,5 | 71,5 | 2,51 |
| Citronensäure-Ester Mono- und Diglycerid (Kokos-Öl basiert, PHL) | Eco Emulsifier 7 | 32,3 | 67,7 | 2,10 |
| Citronensäure-Ester Mono- und Diglycerid (Kokos-Öl basiert, LKA) | Eco Emulsifier 8 | 28,9 | 71,1 | 2,46 |

Dabei liegt das Verhältnis der Anteile der Gesamtmenge an Verbindungen der Formel (I) und einem oder mehrerer Salz(e) Verbindung(en) der Formel (I) mit genau einer durch einen durch eine Ester-Bindung an das Glycerol-Rückgrat gebundenen Fettsäurerest, d.h. MGC, zur Gesamtmenge an Verbindungen der Formel (I) und einem oder mehrerer Salz(e) Verbindung(en) der Formel (I) mit genau zwei durch Ester-Bindungen an das Glycerol-Rückgrat gebundenen Fettsäurereste, d.h. DGC, bezogen auf das Gewicht dieser Verbindungen, im Bereich von 1:1,5 bis 1:3, vorzugsweise im Bereich von 1:1,75 bis 1:2, besonders bevorzugt im Bereich von 1:2 bis 1:2,5.

### Beispiel 5: Darstellung eines erfindungsgemäßen flüssigen Emulgators: Variante 1

In einem beheizten Doppelmantelbehälter mit Blattrührer wurden 900 g (-1.35 mol) Babassu-Öl wie hierin beschrieben mit 189 g (2.05 mol) Glycerin versetzt und auf 50°C gebracht. Eine wässrige Lösung von Natriumhydroxid (10 mol-%) wurde zu dosiert und die Reaktionsmischung auf 250°C erwärmt und bei dieser Temperatur für 120 min gerührt. Entstehendes Prozesswasser wurde durch Anlegen eines Vakuums von 700 mbar entfernt. Die erhaltene Reaktionsmischung wurde abgekühlt, mit Toluol versetzt und mit Wasser extrahiert (zweifach). Die organische Phase wurde abgetrennt und lieferte nach Entfernen des Lösungsmittels das Emulgator-Vorprodukt (1019 g, ~3.6 mol, 89%) mit adäquatem Verhältnis an Mono-, Di- und Triglyceriden.

In einem beheizten Doppelmantelbehälter mit Blattrührer wurden 1019 g (-3.60 mol) Emulgator-Vorprodukt mit Citronensäure (691 g, 3.6 mol) versetzt und unter Rühren auf 155°C für 4h erhitzt. Entstehendes Prozesswasser wurde aus der Reaktionsmischung durch Anlegen eines Vakuums von 800 mbar entfernt. Nach Abkühlen auf Raumtemperatur wurde die Reaktionsmischung mit Toluol und Wasser versetzt, die Phasen getrennt und die organische Phase mit Wasser gewaschen (zweifach) und durch Zugabe einer Lösung von Alkalihydroxid (10 mol-%, NaOH/KOH) neutralisiert. Die organische Phase wurde abgetrennt und lieferte nach Entfernen des Lösungsmittels entsprechend der hierin beschriebenen HPLC-MS-Analyse 990 g eines erfindungsgemäßen Emulgators mit einem Anteil von mehr als 26 Gew.-% an Citronensäureestern und -ethern gemäß den Verbindungen der Formel (I), bezogen auf das Gesamtgewicht des Emulgators. Der erhaltene Emulgator entspricht dem "Eco Emulsifier 4".

### Beispiel 6: Darstellung eines erfindungsgemäßen festen Emulgators: Variante 2

In einem beheizten Doppelmantelbehälter mit Blattrührer wurden 900 g (-1.35 mol) Babassu-Öl wie hierin beschrieben mit 189 g (2.05 mol) Glycerin versetzt und auf 50°C gebracht. Eine wässrige Lösung von Natriumhydroxid (10 mol-%) wurde zu dosiert und die Reaktionsmischung auf 250°C erwärmt und bei dieser Temperatur für 120 min gerührt. Entstehendes Prozesswasser wurde durch Anlegen eines Vakuums von 700 mbar entfernt. Die erhaltene Reaktionsmischung wurde abgekühlt, mit Toluol versetzt und mit Wasser extrahiert (zweifach). Die organische Phase wurde abgetrennt und lieferte nach Entfernen des Lösungsmittels das erfindungsgemäße Emulgator-Vorprodukt (1019 g, -3.6 mol, 89%) mit adäquatem Verhältnis an Mono-, Di- und Triglyceriden.

In einem beheizten Doppelmantelbehälter mit Blattrührer wurden 1019 g (-3.60 mol) Emulgator-Vorprodukt mit Citronensäure (691 g, 3.6 mol) versetzt und unter Rühren auf 155°C für 4h erhitzt. Entstehendes Prozesswasser wurde aus der Reaktionsmischung durch Anlegen eines Vakuums von 800 mbar entfernt. Nach Abkühlen auf Raumtemperatur wurde die Reaktionsmischung mit Toluol und Wasser versetzt, die Phasen getrennt und die organische Phase mit Wasser gewaschen (zweifach) und durch Zugabe einer Lösung von Alkalihydroxid (10 mol-%, NaOH/KOH) neutralisiert. Die Phasen wurden getrennt und die organische Phase vom Lösungsmittel befreit. Der erhaltene Rückstand wurde in 1000 g Alkohol (Methanol, Ethanol, Propanol) aufgenommen und für 2h ausgerührt. Nach Abkühlen und Filtration wurden 260 g eines festen Emulgators mit einem Anteil von mehr als 25 Gew.-% an Citronensäureestern und -ethern erhalten, bezogen auf das Gesamtgewicht des Emulgators. Der nach diesem Verfahren erhaltene Emulgator entspricht dem "Eco Emulsifier 4" (angereichert). Optional konnte durch wiederholte Wäsche mit Methanol/Wasser der Anteil an Citronensäureestern und- ethern gemäß Verbindungen der Formel (I) auf 90 - 100 Gew.-% gesteigert werden, bezogen auf das Gesamtgewicht des Emulgators. Durch Verwendung unterschiedlicher natürlicher Öle wie hierin beschrieben und oben genannter Vorschrift war es möglich, "Eco Emulsifier 5", "Eco Emulsifier 6" und "Eco Emulsifier 7".Anstelle der vorausgehend genannten Verfahrensparameter (Druck, Temperatur, Lösungsmittel, (Stoff)mengenverhältnisse, etc.) führen ebenso weitere Kombinationen von hierin genannten Verfahrensparametern zu erfindungsgemäßen Emulgator-Vorprodukten bzw. Emulgatoren, wobei die einzelnen Verfahrensparameter unabhängig voneinander ausgewählt werden können.

### Anwendungsbeispiel 1: Nachweis der emulgierenden Eigenschaften

Einen direkten Hinweis auf die Effektivität der Emulgierwirkung der erfindungsgemäßen Emulgatoren liefert beispielsweise die volumenbasierte Partikelgrößenbestimmung der Emulsionen mittels Laserbeugung (Messgerät: z.B. Malvern Mastersizer 3000). Kleinere Öltropfen in den erfindungsgemäßen Emulsionen weisen auf eine höhere Reduktion der Oberflächenspannung durch den Emulgator hin. Emulsionen mit kleinen Partikelgrößen sind physikalisch stabiler, zeigen eine verbesserte Sensorik im Sinne bessere Verteilbarkeit auf der Haut sowie ein weißeres und glänzenderes Aussehen.

Verwendet für diese Versuche (s. Tabelle 5) wurde ein Emulgator-Vorprodukt (Versuche Nr. 1 und 5), der Emulgator mit 85 - 100 Gew.-% Aktivgehalt, d.h. mit 85 - 100 Gew.-% der Verbindungen der Formel (I) bezogen auf das Gesamtgewicht des Emulgators, (Versuche Nr. 2 und 6), Kombinationen hieraus (Versuche Nr. 3, 7, 9 und 10) sowie der Emulgator (Versuche Nr. 4 und 8). Ausgangsprodukte für diese Substanzen waren Babassu-Öl bzw. Kokos-Öl. In Versuch Nr. 1 wurde auf erfindungsgemäße Emulgatoren bzw. aus dem Stand der Technik bekannte Emulgatoren verzichtet. Details zur Zusammensetzung der verwendeten Emulgatoren, Vorprodukten, etc. sind weiter unten beschrieben (s. Tabelle 6).

**Tabelle 5 Versuchsaufbau o/w Emulsionen.**

| **Nr.** | **Emulgator / Vorprodukt auf Basis von Kokos-Öl / (w/w%)** | | | **Emulgator / Vorprodukt auf Basis von Babassu-Öl / (w/w%)** | | |
|---|---|---|---|---|---|---|
| | Emulgator-Vorprodukt | Emulgator angereichert | Emulgator | Emulgator-Vorprodukt | Emulgator angereichert | Emulgator |
| 1 | 0,75 | - | - | - | - | - |
| 2 | - | 0,25 | - | - | - | - |
| 3 | 0,75 | 0,25 | - | - | - | - |
| 4 | - | - | 1,0 | - | - | - |
| 5 | - | - | - | 0,75 | - | - |
| 6 | - | - | - | - | 0,25 | - |
| 7 | - | - | - | 0,75 | 0,25 | - |
| 8 | - | - | - | - | - | 1,0 |
| 9 | 0,75 | - | - | - | 0,25 | - |
| 10 | - | 0,25 | - | 0,75 | - | - |
| 11 | - | - | - | - | - | - |

Die Emulsionen Nr. 1 und 5 aus Tabelle 5 bzw. Tabelle 6 zeigten die größten Öltropfen, d.h. die in diesen Formulierungen verwendeten Emulgator-Vorprodukte zeigten im Vergleich zu den weiteren erfindungsgemäßen Emulgatoren eine etwas geringere emulgierende Wirkung. Die Emulsionen Nr. 2, 3, und 4 (Mischungen aus angereichertem Emulgator und dem Emulgator-Vorprodukt bzw. dem Emulgator auf Kokos-Öl Basis) zeigten Öltropfen deutlich geringerer Größe, was auf einen sehr guten emulgierenden Effekt hinweist. Vergleichbar gut sind die Ergebnisse für die Emulsionen Nr. 6, 7 und 8 aus (basierend auf Babassu-Öl). In Versuch Nr. 9 aus wird der angereicherte Emulgator aus Babassu-Öl mit dem Emulgator-Vorprodukt aus Kokos-Öl kombiniert. In Versuch Nr. 10 ist es umgekehrt. Auch in diesen beiden Versuchen zeigte sich der sehr gute emulgierende Effekt durch kleine Öltropfen. Daraus lässt sich schließen, dass die Citrat-Ester für die besonders vorteilhafte emulgierende Wirkung verantwortlich sind.

Die in Tabelle 6 folgenden Rezepturen wurden für die Versuche bzw. O/W-Emulsionen 1 bis 11 aus Anwendungsbeispiel 1 verwendet.

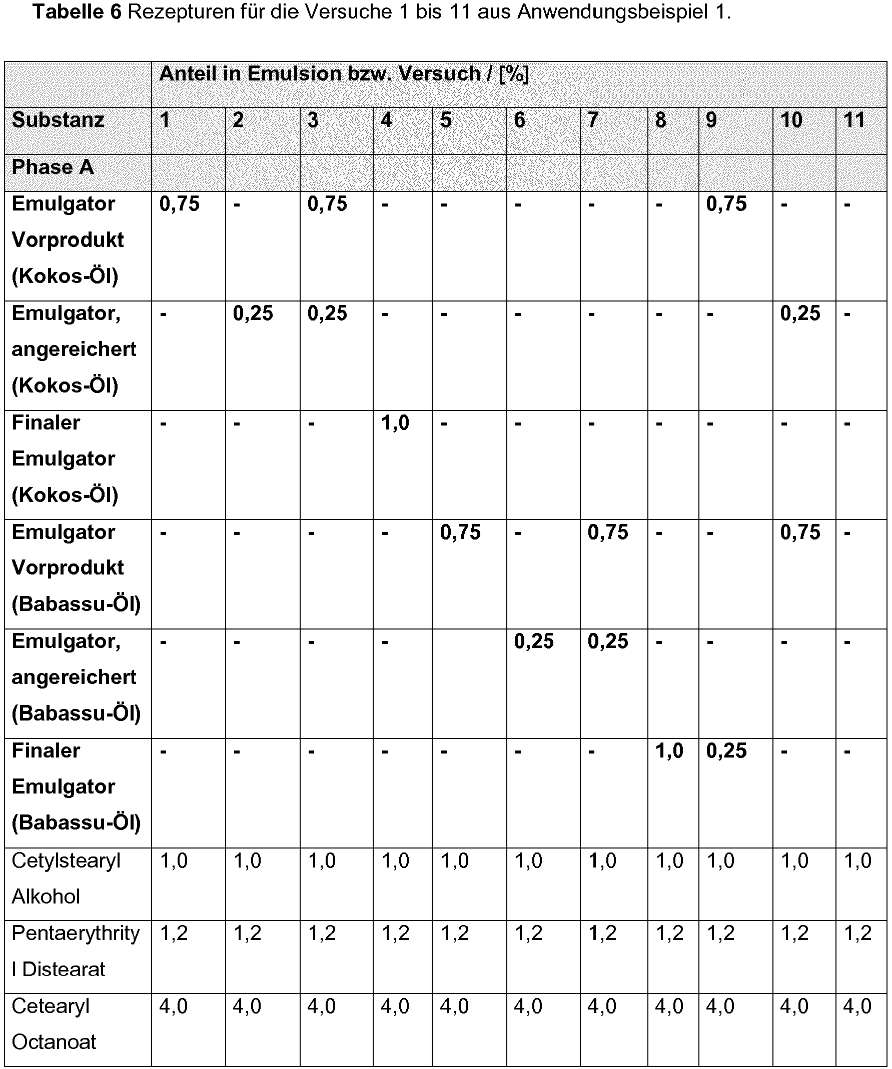

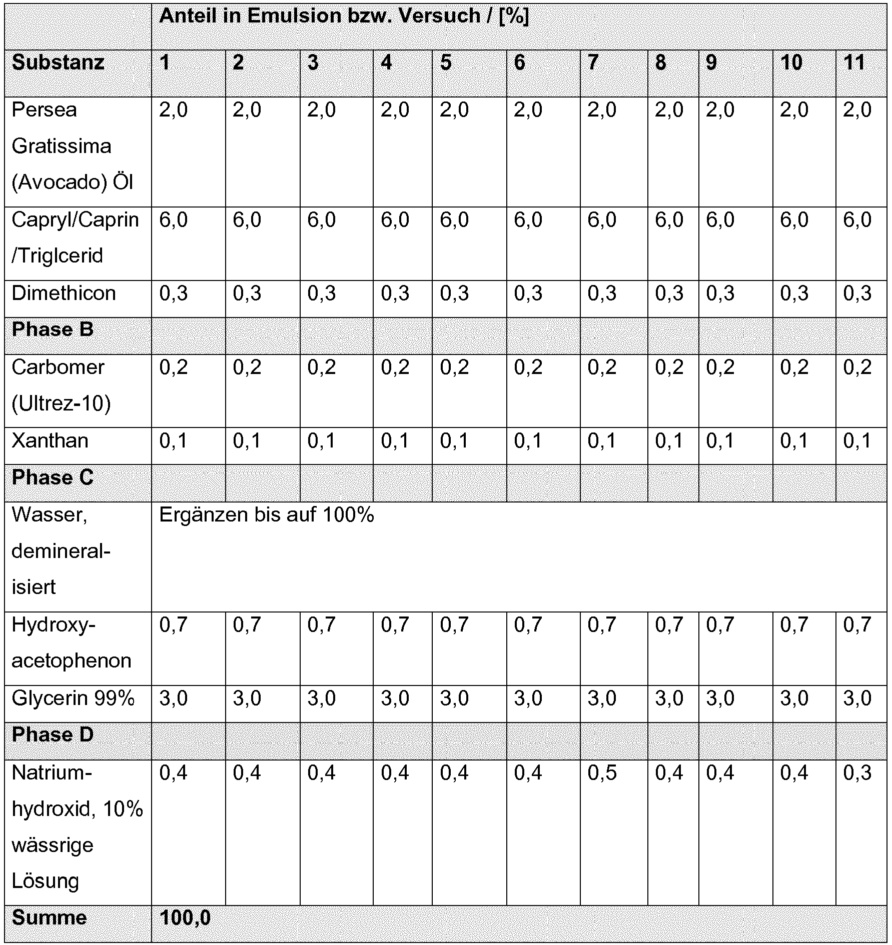

Herstellungshinweis:
- Phase A und C getrennt auf ca. 80°C erwärmen.
- Phase A von der Wärmequelle nehmen, Phase B zugeben und mit Hilfe eines Magnetrührers dispergieren.
- Phase C zu Phase AB geben und emulgieren. (Ultra Turray Rührer, 3 Minuten 6000 U/min).
- Unter Rühren Phase D zugeben und unter Verwendung eines Blattrührers kaltrühren lassen.
- pH-Wert auf 5,8 einstellen.

Erfindungsgemäße Emulgator-Vorprodukte bzw. Emulgatoren (fett gedruckt in "Phase A" von Tabelle 6) können vorzugsweise nach einem der Verfahren der Beispiele 5 oder 6 bzw. deren entsprechenden Analoga hinsichtlich der Verwendung des jeweiligen Ausgangsmaterials (Kokos-Öl bzw. Babassu-Öl) erhalten werden. Zusätzlich können, wie hierin beschrieben, auch andere erfindungsgemäße Kombinationen als von den explizit in den Beispielen 5 und 6 beschriebenen Verfahrensparametern (Druck, Temperatur, Lösungsmittel, (Stoff)mengenverhältnisse, etc.) zu erfindungsgemäßen Emulgator-Vorprodukten bzw. Emulgatoren führen.

Die Ergebnisse der volumenbezogenen Partikelgrößenmessung werden als D_{v 0,5} bzw. als D_{v 0,9} Werte in µm angegeben. Ein D_{v 0,5} von 11,7 µm sagt z.B. aus, dass 50% der Partikel kleiner als 11,7 µm sind, bzw. ein D_{v 0,9} von 23,3 µm, dass 90% der Partikel kleiner als 23,3 µm sind.

Die Ergebnisse der mittels Laserbeugung (Malvern Mastersizer 3000) durchgeführten Partikelgrößenmessung der Emulsionen 1 bis 11 aus Tabelle 6 sind in Tabelle 7 zusammengefasst. Verfahren zur Durchführung von Laserbeugungs-Versuchen sind dem Fachmann hinlänglich bekannt. Vorzugsweise wurden die untersuchten Proben durch einen geeigneten Spritzenfilter filtriert, um Verunreinigungen der Probe (z.B. Staub) zu vermeiden.

**Tabelle 7 Ergebnisse der mittels Laserbeugung durchgeführten Partikelgrößenmessung der Emulsionen 1 bis 11 aus Tabelle 6.**

| Nr. | **D _{v0,5} / [µm]** | **D _{v0,9}/ [µm]** |
|---|---|---|
| 1 | 11,7 | 23,3 |
| 2 | 5,4 | 9,4 |
| 3 | 4,6 | 7,8 |
| 4 | 3,3 | 5,7 |
| 5 | 13,2 | 30,8 |
| 6 | 6,1 | 10,6 |
| 7 | 5,1 | 9,1 |
| 8 | 4,1 | 6,6 |
| 9 | 4,7 | 8,6 |
| 10 | 4,6 | 7,9 |
| 11 | 12,3 | 29,1 |

### Anwendungsbeispiel 2: Emulsionen mit Citrat-Estern mit hohen Anteilen an C12/C14-Fettsäureestern (Citrat Ester Babassu-Öl- bzw. Kokos-Öl-basiert) im Vergleich zu Emulgatoren basierend auf C16/C18 Fettsäuren (Glycerylstearat Citrat/Glyceryloleat Citrat).

Erfindungsgemäße Emulsionen, umfassend erfindungsgemäße Emulgatoren, d.h. Citrat-Ester mit hohen Anteilen an C12- und C14- Fettsäureresten basierend auf Babassu-Öl bzw. Kokos-Öl, werden mit Emulgatoren basierend auf C16- und C18-Fettsäuren (Glycerylstearat Citrat/Glyceryloleat Citrat) verglichen.

Die in Tabelle 8 folgenden Rezepturen wurden für die Versuche bzw. O/W-Emulsionen A bis E aus Anwendungsbeispiel 2 verwendet.

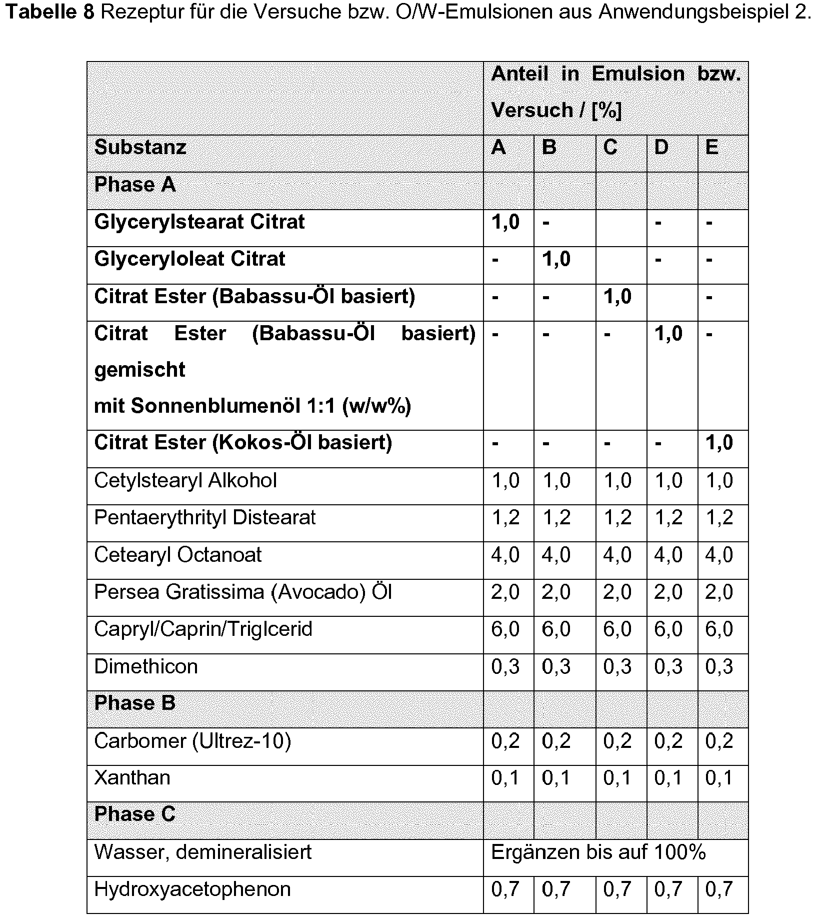

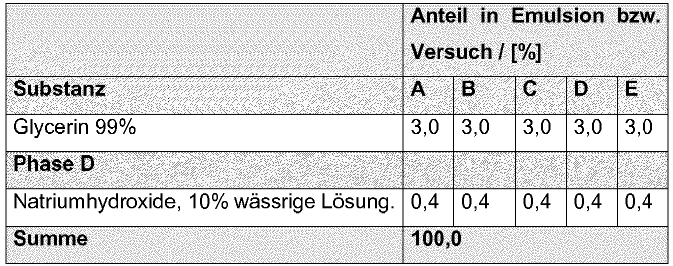

Herstellungshinweis:
- Phase A und C getrennt auf ca. 80°C erwärmen.
- Phase A von der Wärmequelle nehmen, Phase B zugeben und mit Hilfe eines Magnetrührers dispergieren.
- Phase C zu Phase AB geben und emulgieren. (Ultra Turray Rührer, 3 Minuten 6000 U/min).
- Unter Rühren Phase D zugeben und unter Verwendung eines Blattrührers kaltrühren lassen.
- pH-Wert auf 5,8 einstellen.

Die Ergebnisse der mittels Laserbeugung (Malvern Mastersizer 3000) durchgeführten Partikelgrößenmessungen der Emulsionen A bis E aus Tabelle 8 in Tabelle 9 zusammengefasst.

**Tabelle 9 Ergebnisse der mittels Laserbeugung durchgeführten Partikelgrößenmessung der Emulsionen A bis E aus Tabelle 8.**

| **Nr.** | **Emulgator** | **Konz. (%)** | **D _{v0,5} (µm)** | **D _{v0,9} (µm)** |
|---|---|---|---|---|
| A | Glycerylstearat Citrat | 1,0 | 5,9 | 10,7 |
| B | Glyceryloleat Citrat | 1,0 | 5,4 | 9,4 |
| C | Citrat Ester (Babassu-Öl basiert) | 1,0 | 3,9 | 5,9 |
| D | Citrat Ester (Babassu-Öl basiert) gemischt mit Sonnenblumenöl;1:1 (w/w%) | 1,0 | 4,9 | 8,3 |
| E | Citrat Ester (Kokos-Öl) | 1,0 | 3,4 | 5,4 |

Die erfindungsgemäßen Emulsionen C, D und E enthaltend einen oder mehrere erfindungsgemäße Emulgatoren zeigen im Vergleich zu den Emulsionen A und B kleinere Öltropfen. Die erfindungsgemäßen Emulgatoren zeigen demnach eine verbesserte Emulgierwirkung im Vergleich mit herkömmlichen Emulgatoren.

## Patentansprüche

1. Emulgator umfassend oder bestehend aus
einer Mischung aus Verbindungen der Formel (I) 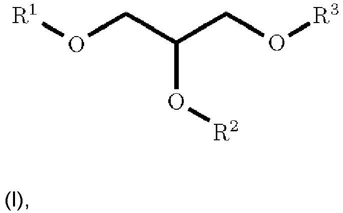
wobei jeweils gilt:
R¹, R² und R³ bedeuten jeweils unabhängig voneinander
(i) ein Wasserstoffatom,
(ii) einen durch eine Ester-Bindung an das Glycerol-Rückgrat gebundenen Fettsäurerest,
oder
(iii) einen durch eine Ester-Bindung an das Glycerol-Rückgrat gebundenen Citronensäurerest,
mit der Maßgabe, dass jeweils höchstens einer der Reste R¹, R² und R³ ein durch eine Ester-Bindung an das Glycerol-Rückgrat gebundener Citronensäurerest (iii) ist,
einem oder mehreren unterschiedlichen Salz(en) der Verbindung(en) der Formel (I) oder einer oder mehreren unterschiedlichen Verbindung(en) der Formel (I) und einem oder mehreren unterschiedlichen Salz(en) der Verbindung(en) der Formel (I),
wobei der Anteil der Gesamtmenge an Verbindungen der Formel (I) und Salzen der Verbindung der Formel (I) mit einem oder zwei durch eine Ester-Bindung an das Glycerol-Rückgrat gebundenen Fettsäurerest(en) (ii) höher als 40 Gew.-%, höher als 50 Gew.-%, höher als 60 Gew.-%, höher als 70 Gew.-%, höher als 80 Gew.-% höher als 90 Gew.-% oder höher als 95 Gew.-% ist, bezogen auf das Gesamtgewicht des Emulgators,
wobei der Anteil der Gesamtmenge an Verbindungen der Formel (I) und Salzen der Verbindungen der Formel (I) mit genau einem durch eine Ester-Bindung an das Glycerol-Rückgrat gebundenen Citronensäurerest (iii) und mindestens einem durch eine Ester-Bindung an das Glycerol-Rückgrat gebundenen Fettsäurerest (ii) höher als 20 Gew.-%, höher als 21 Gew.-%, höher als 22 Gew.-%, höher als 23 Gew.-%, höher als 24 Gew.-% oder als höher als 25 Gew.-% ist, bezogen auf das Gesamtgewicht des Emulgators und,
wobei
der Anteil der Gesamtmenge an Verbindungen der Formel (I) und/oder einem oder mehrerer Salz(en) der Verbindung(en) der Formel (I) mit einem oder mehreren durch eine bzw. mehrere Ester-Bindung(en) an das Glycerol-Rückgrat gebundenen Fettsäurerest(en) (ii), bei denen der bzw. einer, mehrere oder sämtliche Fettsäurerest(e) (ii) unabhängig voneinander ausgewählt ist bzw. sind aus der Gruppe, bestehend aus dem Fettsäurerest der Capronsäure, dem Fettsäurerest der Caprylsäure, dem Fettsäurerest der Caprinsäure, dem Fettsäurerest der Laurinsäure und dem Fettsäurerest der Myristinsäure, höher als 55 Gew.-%, vorzugsweise höher als 70 Gew.-%oder höher als 75 Gew.-%, besonders bevorzugt höher als 90 Gew.-% ist, bezogen auf das Gesamtgewicht der Verbindungen der Formel (I),
und
der Anteil der Gesamtmenge an Verbindungen der Formel (I) und/oder einem oder mehrerer Salz(en) der Verbindung(en) der Formel (I) mit einem oder mehreren durch eine bzw. mehrere Ester-Bindung(en) an das Glycerol-Rückgrat gebundenen Fettsäurerest (ii), bei denen der bzw. einer, mehrere oder sämtliche Fettsäurerest(e) (ii) unabhängig voneinander ausgewählt ist bzw. sind aus der Gruppe, bestehend aus dem Fettsäurerest der Laurinsäure und dem Fettsäurerest der Myristinsäure, höher als 50 Gew.-%, vorzugsweise höher als 55 Gew.-%, besonders bevorzugt höher als 60 Gew.-% ist, bezogen auf das Gesamtgewicht der Verbindungen der Formel (I),
und
wobei für eine oder mehrere Verbindungen der Formel (I) gilt, dass der bzw. die Fettsäurerest(e) (ii) unabhängig voneinander ausgewählt ist bzw. sind aus der Gruppe, bestehend aus dem Fettsäurerest der Palmitinsäure, dem Fettsäurerest der Palmitoleinsäure, dem Fettsäurerest der Stearinsäure, dem Fettsäurerest der Ölsäure, dem Fettsäurerest der Linolsäure und dem Fettsäurerest der Linolensäure, niedriger als 40 Gew.-%, vorzugsweise niedriger als 35 Gew.-%, besonders bevorzugt niedriger als 30 Gew.-% ist, bezogen auf das Gesamtgewicht der Verbindungen der Formel (I).

2. Emulgator gemäß Anspruch 1, wobei die Mischung aus Verbindungen der Formel (I) aus 5 - 20 % Verbindungen der Formel (I) mit genau einem Fettsäurerest und maximal einem Citronensäurerest und aus 20 - 50 % Verbindungen der Formel (I) mit genau zwei Fettsäureresten und maximal einem Citronensäurerest und 25 - 60 % Verbindungen der Formel (I) mit genau drei Fettsäureresten umfasst.

3. Verfahren zur Herstellung eines Emulgators gemäß einem der Ansprüche 1 oder 2, umfassend oder bestehend aus die folgenden Schritte:
(A) Umsetzen einer Fettsäurezusammensetzung bestehend aus oder umfassend eine oder mehrere Fettsäuren und/oder einen oder mehrere Fettsäurereste,
wobei die bzw. eine, mehrere oder sämtliche Fettsäure(n) ausgewählt ist bzw. sind aus der Gruppe, bestehend aus Capronsäure, Caprylsäure, Caprinsäure, Laurinsäure, Myristinsäure, Palmitinsäure, Palmitoleinsäure, Stearinsäure, Ölsäure, Linolsäure und Linolensäure
und/oder einen oder mehrere Fettsäurereste,
wobei der bzw. ein, mehrere oder sämtliche Fettsäurerest(e) (ii) ausgewählt ist bzw. sind aus der Gruppe, bestehend aus dem Fettsäurerest der Capronsäure, Fettsäurerest der Caprylsäure, Fettsäurerest der Caprinsäure, Fettsäurerest der Laurinsäure, Fettsäurerest der Myristinsäure, Fettsäurerest der Palmitinsäure, Fettsäurerest der Palmitoleinsäure, Fettsäurerest der Stearinsäure, Fettsäurerest der Ölsäure, Fettsäurerest der Linolsäure und Fettsäurerest der Linolensäure, umfassend wenigstens Laurinsäure und Myristinsäure und/oder den Fettsäurerest der Laurinsäure und/oder den Fettsäurerest der Myristinsäure,
wobei die Fettsäurezusammensetzung ausgewählt ist aus der Gruppe bestehend aus einem Öl, einem Fett, einem fetten Öl oder Mischungen daraus oder umfassend solche,
mit Glycerol
und
einer oder mehreren katalytischen Basen,
(B) Zugeben von Citronensäure, einem oder mehrerer Salz(e) der Citronensäure oder einer Mischung aus Citronensäure und einem oder mehreren Salz(en) der Citronensäure, zu der in Schritt (A) erhaltenen Mischung,
und
(C) optional: Fällen der in Schritt (B) erhaltenen Mischung mit einem oder mehreren Lösungsmitteln zur Anreicherung der Verbindung(en) der Formel (I),
und
(D) optional:
Aufreinigen der aus Schritt (B) oder (C) erhaltenen Mischung unter Verwendung eines Lösungsmittels oder mehrerer Lösungsmittel ausgewählt aus der Gruppe, bestehend aus halogenierten, polaren, protisch polaren, protisch unpolaren, "grünen" Lösungsmitteln und Mischungen daraus oder umfassend solche,
und/oder
Durchführen eines Aufreinigungsverfahrens oder mehrerer Aufreinigungsverfahren ausgewählt aus der Gruppe, bestehend aus Filtration, Ultra- oder Nanofiltration, Ad- und Absorbtionstechniken, Extraktion, Umkristallisation und chromatographischen Methoden und Kombinationen daraus,
wobei in der in Schritt (A) eingesetzten Fettsäurezusammensetzung der Anteil der Gesamtmenge an Fettsäuren aus der Gruppe bestehend aus Capronsäure, Caprylsäure, Caprinsäure, Laurinsäure und Myristinsäure und/oder Fettsäureresten aus der Gruppe bestehend aus dem Fettsäurereste der Capronsäure, dem Fettsäurereste der Caprylsäure, dem Fettsäurereste der Caprinsäure, dem Fettsäurereste der Laurinsäure und dem Fettsäurereste der Myristinsäure, höher als 45 Gew.-%, vorzugsweise höher als 55 Gew.-%, ist, bezogen auf das Gesamtgewicht der Fettsäurezusammensetzung, und
wobei in der in Schritt (A) eingesetzten Fettsäurezusammensetzung der Anteil der Gesamtmenge an
- Laurinsäure und/oder des Fettsäurerests der Laurinsäure höher als 20 Gew.-%, vorzugsweise höher als 35 Gew.-%, besonders bevorzugt höher als 40 Gew.-% ist, vorzugsweise, bezogen auf das Gesamtgewicht der Fettsäurezusammensetzung, und/oder
- Myristinsäure und/oder des Fettsäurerests der Myristinsäure höher als 5 Gew.-%, vorzugsweise höher als 10 Gew.-%, besonders bevorzugt höher als 15 Gew.-% ist, bezogen auf das Gesamtgewicht der Fettsäurezusammensetzung.

4. Verfahren gemäß Anspruch 3, wobei in Schritt (A) Glycerol zum Umsetzen in einer Menge von 0,5 bis 5 mol-Äquivalenten, vorzugsweise in einer Menge von 1,0 bis 2,0 mol-Äquivalenten, besonders bevorzugt in einer Menge von 1,25 bis 1,75 mol-Äquivalenten eingesetzt wird, bezogen auf die gesamte Stoffmenge Fettsäurezusammensetzung, und/oder
wobei die bzw. eine, mehrere oder sämtliche katalytische Base(n) aus Schritt (A) in einer Menge von 0,01 bis 0,5 mol-Äquivalenten, vorzugsweise in einer Menge von 0,025 bis 0,1 mol-Äquivalenten, besonders bevorzugt in einer Menge von 0,05 bis 0,1 mol-Äquivalenten eingesetzt wird / werden, bezogen auf die gesamte Stoffmenge der Fettsäurezusammensetzung
und/oder
wobei die bzw. eine, mehrere oder sämtliche katalytische Base(n) aus Schritt (A) unabhängig voneinander ausgewählt ist bzw. sind aus der Gruppe, bestehend aus Aminbasen, Aminosäuren, Brønsted Basen, Lewis Basen, Alkalisalzen, Erdalkalisalzen und Mischungen daraus, vorzugsweise aus der Gruppe, bestehend aus Natriumhydroxid, Kaliumhydroxid und Mischungen daraus.

5. Verfahren gemäß einem der Ansprüche 3 bis 4, wobei Schritt (A) bei einer Temperatur im Bereich von 150 bis 300°C, vorzugsweise im Bereich von 175 bis 275°C, besonders bevorzugt im Bereich von 200 bis 250°C,
und optional unter Vakuum-Bedingungen
durchgeführt wird,
und/oder
wobei in Schritt (B) eine Gesamtmenge an Citronensäure und/oder einem oder mehreren Salz(en) davon im Bereich von 0,1 bis 2,0 Gewichtsteilen, vorzugsweise im Bereich von 0,25 bis 1,5 Gewichtsteilen, besonders bevorzugt im Bereich von 0,25 bis 1,0 Gewichtsteilen zugegeben wird, bezogen auf das Gesamtgewicht der in Schritt (A) erhaltenen Mischung.

6. Verfahren gemäß einem der Ansprüche 3 bis 5, wobei für die in Schritt (B) nach erfolgter Zugabe von Citronensäure und/oder einem oder mehreren Salz(en) davon ablaufende Reaktion eine Temperatur im Bereich von 50 bis 200°C, vorzugsweise im Bereich von 100 bis 175°C, besonders bevorzugt im Bereich von 120 bis 160°C
und optional Vakuum-Bedingungen
eingestellt wird bzw. werden.

7. Verfahren einem der Ansprüche 3 bis 6, wobei das bzw. ein, mehrere oder sämtliche Lösungsmittel in Schritt (C), falls vorhanden, bevorzugt ausgewählt ist bzw. sind aus der Gruppe, bestehend aus Pentan, Hexan, Heptan, Cyclopentan, Cyclohexan, Tetrachlormethan, Diisopropylether, Toluol, Benzol, Diethylether, Dichlormethan, Chloroform, Aceton, Dioxan, Tetrahydrofuran, 2-Methylthetrahydrofuran, tert-Butylmethylether, Ethylacetat, Dimethylsulfoxid, Acetonitril, Benzonitril, Pyridin, 2-Propanol, Ethanol, Methanol, Essigsäure, Ameisensäure, Wasser und Mischungen daraus,
und/oder
wobei die Fettsäurezusammensetzung ausgewählt ist aus der Gruppe, bestehend aus Babassu-Öl, Palmkern-Öl, Kokos-Öl, Macaúba-Öl, Mikroalgen-Öl, Neutralöl und Mischungen daraus.

8. Emulgator-Vorprodukt, erhalten durch oder erhältlich durch Aufreinigen der aus Schritt (B) gemäß einem der Ansprüche 3 bis 7 erhaltenen Mischung
unter Verwendung eines Lösungsmittels oder mehrerer Lösungsmittel ausgewählt aus der Gruppe, bestehend aus halogenierten, polaren, protisch polaren, protisch unpolaren, "grünen" Lösungsmitteln und Mischungen daraus,
und/oder
durch Durchführen eines Aufreinigungsverfahrens oder mehrerer Aufreinigungsverfahren ausgewählt aus der Gruppe, bestehend aus Filtration, Ultra- oder Nanofiltration, Ad- und Absorbtionstechniken, Extraktion, Umkristallisation und chromatographischen Methoden und Kombinationen daraus.

9. Emulgator gemäß einem der Ansprüche 1 bis 2, erhalten durch oder erhältlich durch ein Verfahren gemäß einem der Ansprüche 3 bis 7, vorzugsweise
erhalten durch oder erhältlich durch ein Verfahren gemäß einem der Ansprüche 3 bis 8,
wobei das Verfahren Schritt (C) umfasst,
und optional wobei der Anteil der Gesamtmenge an Verbindungen der Formel (I) und einem oder mehrerer Salz(e) der Verbindung(en) der Formel (I) mit genau einem durch eine Ester-Bindung an das Glycerol-Rückgrat gebundenen Citronensäurerest (iii) höher als 50 Gew.-%, vorzugsweise höher als 85 Gew.-%, ist, bezogen auf das Gesamtgewicht des Emulgators, und vorzugsweise wobei das Verhältnis der Anteile der Gesamtmenge an Verbindungen der Formel (I) und einem oder mehrerer Salz(e) Verbindung(en) der Formel (I) mit genau einer durch einen durch eine Ester-Bindung an das Glycerol-Rückgrat gebundenen Fettsäurerest zur Gesamtmenge an Verbindungen der Formel (I) und einem oder mehrerer Salz(e) Verbindung(en) der Formel (I) mit genau zwei durch EsterBindungen an das Glycerol-Rückgrat gebundenen Fettsäurereste im Bereich von 1:1,5 bis 1:3, vorzugsweise im Bereich von 1:1,75 bis 1:2, besonders bevorzugt im Bereich von 1:2 bis 1:2,5 liegt, bezogen auf das Gewicht dieser Verbindungen.

10. Verwendung einer Mischung aus Verbindung(en) der Formel (I) wie in einem der Ansprüche 1 oder 2 definiert als Emulgator.

11. Emulsion umfassend oder bestehend aus
- einer Öl-Phase, enthaltend ein oder mehrere Emulgator-Vorprodukt(e) gemäß Anspruch 8 und/oder einen oder mehrere Emulgator(en) gemäß einem der Ansprüche 1, 2 oder 9 oder eine Mischung daraus,
und
- einer wässrigen Phase,
vorzugsweise zudem umfassend eine oder mehrere unterschiedliche Verbindung(en) zur Senkung oder Erhöhung der Viskosität der Emulsion.

12. Verwendung einer Emulsion gemäß Anspruch 11 in einer der Reinigung dienenden Zubereitung, einer kosmetischen oder pharmazeutischen, vorzugsweise einer dermatologischen, Zubereitung, einer dem Genuss oder der Ernährung dienenden Zubereitung oder in einer Halbfertigware zur Herstellung einer solchen Zubereitung.

13. Der Reinigung dienende Zubereitung, kosmetische oder pharmazeutische, vorzugsweise dermatologische, Zubereitung, dem Genuss oder der Ernährung dienende Zubereitung oder Halbfertigware, umfassend ein oder mehrere Emulgator-Vorprodukt(e) gemäß Anspruch 8 oder einen oder mehrere Emulgator(en) gemäß einem der Ansprüche 1, 2 oder 9 oder eine oder mehrere Emulsion(en) gemäß Anspruch 11.

14. Verfahren zur Herstellung einer Emulsion gemäß Anspruch 11, umfassend oder bestehend aus den folgenden Schritten:
- Bereitstellen
einer Öl-Phase, enthaltend ein Emulgator-Vorprodukt gemäß Anspruch 8 oder einen Emulgator gemäß einem der Ansprüche 1, 2 oder 9 oder eine Mischung daraus,
und
einer wässrigen Phase,
- optional: getrenntes Erwärmen der Öl-Phase und der wässrigen Phase, vorzugsweise auf eine Temperatur im Bereich von 40 bis 100°C, vorzugsweise im Bereich von 60 bis 85°C,
- optional: Zugeben einer oder mehrerer unterschiedlicher Verbindungen zur Senkung oder Erhöhung der Viskosität zur erwärmten Öl-Phase,
- Mischen der optional erwärmten wässrigen Phase und der erwärmten Öl-Phase, und
- optional: Abkühlen unter Rühren.

## Claims

1. Emulsifier comprising or consisting of
a mixture of compounds of formula (I) 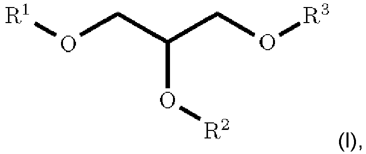
wherein each of the following applies:
R¹, R² and R³ each mean independently of each other
(i) a hydrogen atom,
(ii) a fatty acid residue attached to the glycerol backbone by an ester linkage,
or
(iii) a citric acid residue bound to the glycerol backbone by a ester linkage,
with the proviso that in each case at most one of the residues R¹, R² and R³ is a citric acid residue (iii) bonded to the glycerol backbone by an ester linkage,
one or more different salt(s) of the compound(s) of formula (I), or one or more different compound(s) of formula (I) and one or more different salt(s) of the compound(s) of formula (I),
wherein the proportion of the total amount of compounds of formula (I) and salts of the compound of formula (I) having one or two fatty acid residues (ii) attached to the glycerol backbone by an ester linkage is higher than 40 % by weight, higher than 50 % by weight, higher than 60 % by weight, higher than 70 % by weight, higher than 80 % by weight, higher than 90 % by weight or higher than 95 % by weight, based on the total weight of the emulsifier,
wherein the proportion of the total amount of compounds of formula (I) and salts of the compounds of formula (I) having exactly one citric acid residue (iii) bound to the glycerol backbone by an ester linkage and at least one fatty acid residue (ii) bound to the glycerol backbone by an ester linkage is higher than 20 wt.% by weight, higher than 21 % by weight, higher than 22 % by weight, higher than 23 % by weight, higher than 24 % by weight or higher than 25 % by weight, based on the total weight of the emulsifier and,
wherein
the proportion of the total amount of compounds of formula (I) and/or one or more salt(s) of the compound(s) of formula (I) having one or more fatty acid residue(s) (ii) attached to the glycerol backbone by one or more ester linkage(s), wherein one, more or all fatty acid residue(s) (ii) is/are independently selected from the group consisting of the fatty acid residue of caproic acid, the fatty acid residue of caprylic acid, the fatty acid residue of capric acid, the fatty acid residue of lauric acid and the fatty acid residue of myristic acid, higher than 55 wt.% by weight, preferably higher than 70 % by weight or higher than 75 % by weight, particularly preferably higher than 90 % by weight, based on the total weight of the compounds of formula (I),
and
the proportion of the total amount of compounds of formula (I) and/or one or more salt(s) of the compound(s) of formula (I) having one or more fatty acid residue(s) (ii) attached to the glycerol backbone by one or more ester linkage(s), in which one, more or all fatty acid residue(s) (ii) is/are independently selected from the group consisting of the fatty acid residue of lauric acid and the fatty acid residue of myristic acid, is higher than 50 wt.% by weight, preferably higher than 55 % by weight, particularly preferably higher than 60 % by weight, based on the total weight of the compounds of formula (I),
and
wherein, for one or more compounds of formula (I), the fatty acid residue(s) (ii) is/are independently selected from the group consisting of the fatty acid residue of palmitic acid, the fatty acid residue of palmitoleic acid, the fatty acid residue of stearic acid, the fatty acid residue of oleic acid, the fatty acid residue of linoleic acid and the fatty acid residue of linolenic acid, is lower than 40% by weight, preferably lower than 35% by weight, more preferably lower than 30% by weight, based on the total weight of the compounds of formula (I).% by weight, preferably less than 35 % by weight, particularly preferably less than 30 % by weight, based on the total weight of the compounds of formula (I).

2. Emulsifier according to claim 1, wherein the mixture of compounds of formula (I) comprises 5 - 20 % of compounds of formula (I) with exactly one fatty acid residue and at most one citric acid residue and 20 - 50 % of compounds of formula (I) with exactly two fatty acid residues and at most one citric acid residue and 25 - 60 % of compounds of formula (I) with exactly three fatty acid residues.

3. Process for producing an emulsifier according to any one of claims 1 or 2, comprising or consisting of the following steps:
(A) reacting a fatty acid composition consisting of or comprising one or more fatty acids and/or one or more fatty acid residues,
wherein one, more or all fatty acid(s) is/are selected from the group consisting of caproic acid, caprylic acid, capric acid, lauric acid, myristic acid, palmitic acid, palmitoleic acid, stearic acid, oleic acid, linoleic acid and linolenic acid
and/or one or more fatty acid residues,
wherein one, more or all fatty acid residue(s) (ii) is/are selected from the group consisting of the fatty acid residue of caproic acid, fatty acid residue of caprylic acid, fatty acid residue of capric acid, fatty acid residue of lauric acid, fatty acid residue of myristic acid, fatty acid residue of palmitic acid, fatty acid residue of palmitoleic acid, fatty acid residue of stearic acid, fatty acid residue of oleic acid, fatty acid residue of linoleic acid and fatty acid residue of linolenic acid, comprising at least lauric acid and myristic acid and/or the fatty acid residue of lauric acid and/or the fatty acid residue of myristic acid,
wherein the fatty acid composition is selected from the group consisting of, or comprises, an oil, a fat, a fatty oil or mixtures thereof,
with glycerol
and
one or more catalytic bases,
(B) adding citric acid, one or more salt(s) of citric acid or a mixture of citric acid and one or more salt(s) of citric acid, to the mixture obtained in step (A),
and
(C) optionally: precipitating the mixture obtained in step (B) with one or more solvents to enrich the compound(s) of formula (I),
and
(D) optionally:
purifying the mixture obtained from step (B) or (C) using a solvent or more solvents selected from the group consisting of, or comprising, halogenated, polar, protic polar, protic non-polar, "green" solvents and mixtures thereof,
and/or
performing one or more purification processes selected from the group consisting of filtration, ultrafiltration or nanofiltration, adsorption and absorption techniques, extraction, recrystallization and chromatographic methods and combinations thereof,
wherein in the fatty acid composition used in step (A), the proportion of the total amount of fatty acids selected from the group consisting of caproic acid, caprylic acid, capric acid, lauric acid and myristic acid and/or fatty acid residues selected from the group consisting of the fatty acid residue of caproic acid, the fatty acid residue of caprylic acid, the fatty acid residue of capric acid, the fatty acid residue of lauric acid and the fatty acid residue of myristic acid is higher than 45 % by weight, preferably higher than 55 % by weight, based on the total weight of the fatty acid composition, and%, preferably higher than 55 % by weight, based on the total weight of the fatty acid composition, and
wherein in the fatty acid composition used in step (A), the proportion of the total amount of
- lauric acid and/or the fatty acid residue of lauric acid is higher than 20% by weight, preferably higher than 35% by weight, particularly preferably higher than 40% by weight, preferably based on the total weight of the fatty acid composition,
and/or
- myristic acid and/or the fatty acid residue of myristic acid is higher than 5% by weight, preferably higher than 10% by weight, particularly preferably higher than 15% by weight, based on the total weight of the fatty acid composition.

4. Process according to claim 3, wherein in step (A) glycerol is used for reacting in an amount of 0.5 to 5 mol equivalents, preferably in an amount of 1.0 to 2.0 mol equivalents, particularly preferably in an amount of 1.25 to 1.75 mol equivalents, based on the total amount of fatty acid composition,
and/or
wherein one, more or all catalytic base(s) from step (A) is/are used in an amount of from 0.01 to 0.5 mol equivalents, preferably in an amount of from 0.025 to 0.1 mol equivalents, particularly preferably in an amount of from 0.05 to 0.1 mol equivalents, based on the total molar amount of the fatty acid composition
and/or
wherein one, more or all catalytic base(s) of step (A) is/are independently selected from the group consisting of amine bases, amino acids, Brønsted bases, Lewis bases, alkali metal salts, alkaline earth metal salts and mixtures thereof, preferably from the group consisting of sodium hydroxide, potassium hydroxide and mixtures thereof.

5. Process according to any one of claims 3 to 4, wherein step (A) is carried out at a temperature ranging from 150 to 300°C, preferably ranging from 175 to 275°C, more preferably ranging from 200 to 250°C,
and optionally under vacuum conditions,
and/or
wherein in step (B) a total amount of citric acid and/or one or more salt(s) thereof ranging from 0.1 to 2.0 parts by weight, preferably ranging from 0.25 to 1.5 parts by weight, more preferably ranging from 0.25 to 1.0 parts by weight, is added, based on the total weight of the mixture obtained in step (A).

6. Process according to any one of claims 3 to 5, wherein for the reaction taking place in step (B) after the addition of citric acid and/or one or more salt(s) thereof a temperature ranging from 50 to 200°C, preferably ranging from 100 to 175°C, particularly preferably ranging from 120 to 160°C
and optional vacuum conditions
is/are set.

7. Process according to any one of claims 3 to 6, wherein one or more or all solvents in step (C), if present, are preferably selected from the group consisting of pentane, hexane, heptane, cyclopentane, cyclohexane, tetrachloromethane, diisopropyl ether, toluene, benzene, diethyl ether, dichloromethane, chloroform, acetone, dioxane, tetrahydrofuran, 2-methylthetrahydrofuran, tert-butyl methyl ether, ethyl acetate, dimethyl sulphoxide, acetonitrile, benzonitrile, pyridine, 2-propanol, ethanol, methanol, acetic acid, formic acid, water and mixtures thereof,
and/or
wherein the fatty acid composition is selected from the group consisting of babassu oil, palm kernel oil, coconut oil, macaúba oil, microalgae oil, neutral oil and mixtures thereof.

8. Emulsifier precursor obtained by or obtainable by purification of the mixture obtained from step (B) according to any one of claims 3 to 7
using one or more solvents selected from the group consisting of halogenated, polar, protic polar, protic non-polar, "green" solvents and mixtures thereof,
and/or
by carrying out one or more purification processes selected from the group consisting of filtration, ultrafiltration or nanofiltration, adsorption and absorption techniques, extraction, recrystallization and chromatographic methods and combinations thereof.

9. Emulsifier according to any one of claims 1 to 2, obtained by or obtainable by a process according to any one of claims 3 to 7, preferably
obtained by or obtainable by a process according to any one of claims 3 to 8,
wherein the method comprises step (C),
and optionally wherein the proportion of the total amount of compounds of formula (I) and one or more salt(s) of the compound(s) of formula (I) having exactly one citric acid residue (iii) attached to the glycerol backbone by an ester linkage is higher than 50 wt.%, preferably higher than 85 wt.-%, based on the total weight of the emulsifier, and preferably wherein the ratio of the proportions of the total amount of compounds of formula (I) and one or more salt(s) compound(s) of formula (I) having exactly one fatty acid residue attached to the glycerol backbone by an ester linkage to the total amount of compounds of formula (I) and one or more salt(s) of compound(s) of formula (I) having exactly two fatty acid residues attached to the glycerol backbone by ester linkages is in the range from 1:1.5 to 1:3, preferably in the range from 1:1.75 to 1:2, particularly preferably in the range from 1:2 to 1:2.5, based on the weight of these compounds.

10. Use of a mixture of compound(s) of formula (I) as defined in any one of claims 1 or 2 as an emulsifier.

11. Emulsion comprising or consisting of
- an oil phase comprising one or more emulsifier precursor(s) according to claim 8 and/or one or more emulsifier(s) according to any one of claims 1, 2 or 9 or a mixture thereof,
and
- an aqueous phase,
preferably further comprising one or more different compound(s) for reducing or increasing the viscosity of the emulsion.

12. Use of an emulsion according to claim 11 in a preparation for cleaning, a cosmetic or pharmaceutical, preferably dermatological, preparation, a preparation for consumption or nutrition or in a semi-finished product for the production of such a preparation.

13. Cleaning preparation, cosmetic or pharmaceutical, preferably dermatological, preparation, preparation for consumption or nutrition or semi-finished product, comprising one or more emulsifier precursor(s) according to claim 8 or one or more emulsifier(s) according to any of claims 1, 2 or 9 or one or more emulsion(s) according to claim 11.

14. Process for producing an emulsion according to claim 11, comprising or consisting of the following steps:
- providing
an oil phase comprising an emulsifier precursor according to claim 8 or an emulsifier according to any one of claims 1, 2 or 9 or a mixture thereof,
and
an aqueous phase,
- optionally: separately heating the oil phase and the aqueous phase, preferably to a temperature in the range from 40 to 100°C, preferably in the range from 60 to 85°C,
- optionally: adding one or more different compounds to reduce or increase the viscosity of the heated oil phase,
- mixing the optionally heated aqueous phase and the heated oil phase, and
- optionally: cooling while stirring.

## Revendications

1. Émulsifiant comprenant ou constitué de
un mélange de composés de formule (I) 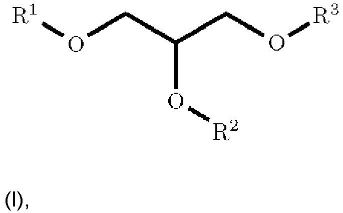
dans laquelle s'applique respectivement:
R¹, R² et R³ signifient chacun indépendamment l'un de l'autre
(i) un atome d'hydrogène,
(ii) un résidu d'acide gras lié au squelette glycérol par une liaison ester,
ou
(iii) un résidu d'acide citrique lié au squelette glycérol par une liaison ester,
à condition que respectivement tout au plus un des résidus R¹, R² et R³ soit un résidu d'acide citrique (iii) lié au squelette glycérol par une liaison ester,
un ou plusieurs sel(s) différent(s) du ou des composé(s) de formule (I) ou un ou plusieurs composé(s) différent(s) de formule (I) et un ou plusieurs sel(s) différent(s) du ou des composé(s) de la formule (I),
dans lequel la proportion de la quantité totale de composés de formule (I) et de sels du composé de formule (I) avec un ou deux résidu(s) d'acide gras (ii) lié(s) au squelette glycérol par une liaison ester est supérieure à 40 % en poids, supérieure à 50 % en poids, supérieure à 60 % en poids, supérieure à 70 % en poids, supérieure à 80 % en poids, supérieure à 90 % en poids ou supérieure à 95 % en poids, par rapport au poids total de l'émulsifiant,
dans lequel la proportion de la quantité totale de composés de formule (I) et de sels des composés de formule (I) avec exactement un résidu d'acide citrique (iii) lié au squelette glycérol par une liaison ester et au moins un résidu d'acide gras (ii) lié au squelette glycérol par une liaison ester est supérieure à 20 % en poids, supérieure à 21 % en poids, supérieure à 22 % en poids, supérieure à 23 % en poids, supérieure à 24 % en poids ou supérieure à 25 % en poids, par rapport au poids total de l'émulsifiant, et
dans lequel
la proportion de la quantité totale de composés de formule (I) et/ou d'un ou de plusieurs sel(s) du ou des composé(s) de formule (I) avec un ou plusieurs résidu(s) d'acide gras (ii) lié(s) au squelette glycérol par une ou bien plusieurs liaison(s) ester, dans lesquels le ou bien un, plusieurs ou tous les résidus d'acide gras (ii) est ou bien sont choisi(s) indépendamment les uns des autres dans le groupe constitué du résidu d'acide gras de l'acide caproïque, du résidu d'acide gras de l'acide caprylique, du résidu d'acide gras de l'acide caprique, du résidu d'acide gras de l'acide laurique et du résidu d'acide gras de l'acide myristique, est supérieure à 55 % en poids, de préférence supérieure à 70 % en poids ou supérieure à 75 % en poids, de manière particulièrement préférée supérieure à 90 % en poids, par rapport au poids total des composés de formule (I),
et
la proportion de la quantité totale de composés de formule (I) et/ou d'un ou de plusieurs sel(s) du ou des composé(s) de formule (I) avec un ou plusieurs résidu(s) d'acide gras (ii) lié(s) au squelette glycérol par une ou bien plusieurs liaison(s) ester, dans lesquels le ou bien un, plusieurs ou tous les résidu(s) d'acide gras (ii) est ou bien sont choisi(s) indépendamment les uns des autres dans le groupe constitué du résidu d'acide gras de l'acide laurique et du résidu d'acide gras de l'acide myristique, est supérieure à 50 % en poids, de préférence supérieure à 55 % en poids, de manière particulièrement préférée supérieure à 60 % en poids, par rapport au poids total des composés de formule (I),
et
dans lequel il s'applique pour un ou plusieurs composés de formule (I) que le ou les résidu(s) d'acide gras (ii) soit ou bien soient choisi(s) indépendamment les uns des autres dans le groupe constitué du résidu d'acide gras de l'acide palmitique, du résidu d'acide gras de l'acide palmitoléique, du résidu d'acide gras de l'acide stéarique, du résidu d'acide gras de l'acide oléique, du résidu d'acide gras de l'acide linoléique et du résidu d'acide gras de l'acide linolénique, est inférieur à 40 % en poids, de préférence inférieur à 35 % en poids, de manière particulièrement préférée inférieur à 30 % en poids, par rapport au poids total des composés de formule (I).

2. Émulsifiant selon la revendication 1, dans lequel le mélange de composés de formule (I) comprenant 5 à 20 % de composés de formule (I) avec exactement un résidu d'acide gras et au maximum un résidu d'acide citrique et de 20 à 50 % de composés de formule (I) avec exactement deux résidus d'acide gras et au maximum un résidu d'acide citrique et 25 à 60 % de composés de formule (I) avec exactement trois résidus d'acide gras.

3. Procédé de fabrication d'un émulsifiant selon l'une quelconque des revendications 1 ou 2, comprenant ou consistant en les étapes suivantes:
(A) faire réagir une composition d'acides gras constituée de ou comprenant un ou plusieurs acides gras et/ou un ou plusieurs résidus d'acide gras,
dans lequel le ou bien un, plusieurs ou tous les acide(s) gras est ou bien sont choisi(s) dans le groupe constitué de l'acide caproïque, de l'acide caprylique, de l'acide caprique, de l'acide laurique, de l'acide myristique, de l'acide palmitique, de l'acide palmitoléique, de l'acide stéarique, de l'acide oléique, de l'acide linoléique et de l'acide linolénique
et/ou un ou plusieurs résidus d'acide gras,
dans lequel le ou bien un, plusieurs ou tous les résidu(s) d'acide gras (ii) est ou bien sont choisi(s) dans le groupe constitué du résidu d'acide gras de l'acide caproïque, du résidu d'acide gras de l'acide caprylique, du résidu d'acide gras de l'acide caprique, du résidu d'acide gras d'acide laurique, du résidu d'acide gras de l'acide myristique, du résidu d'acide gras de l'acide palmitique, du résidu d'acide gras de l'acide palmitoléique, du résidu d'acide gras de l'acide stéarique, du résidu d'acide gras de l'acide oléique, du résidu d'acide gras de l'acide linoléique et du résidu d'acide gras de l'acide linolénique, comprenant au moins l'acide laurique et l'acide myristique et/ou le résidu d'acide gras de l'acide laurique et/ou le résidu d'acide gras de l'acide myristique,
dans lequel la composition d'acides gras est choisie dans le groupe constitué d'une huile, d'une graisse, d'une huile grasse ou de mélanges de celles-ci ou comprenant de telles,
avec du glycérol
et
une ou plusieurs bases catalytiques,
(B) ajouter de l'acide citrique, un ou plusieurs sel(s) de l'acide citrique ou un mélange de l'acide citrique et d'un ou de plusieurs sel(s) de l'acide citrique au mélange obtenu à l'étape (A),
et
(C) en option: précipiter le mélange obtenu à l'étape (B) avec un ou plusieurs solvants pour enrichir le(s) composé(s) de formule (I),
et
(D) en option:
purifier le mélange obtenu à l'étape (B) ou (C) en utilisant un solvant ou plusieurs solvants choisi(s) dans le groupe constitué de solvants halogénés, polaires, protiques polaires, protiques non polaires, "verts" et de mélanges de ceux-ci ou comprenant de tels solvants,
et/ou
mettre en oeuvre un procédé de purification ou plusieurs procédés de purification choisi(s) dans le groupe constitué par la filtration, l'ultra- ou la nanofiltration, les techniques d'adsorption et d'absorption, l'extraction, la recristallisation et les méthodes de chromatographie et leurs combinaisons,
dans lequel dans la composition d'acides gras utilisée à l'étape (A), la proportion de la quantité totale d'acides gras du groupe constitué de l'acide caproïque, de l'acide caprylique, de l'acide caprique, de l'acide laurique et de l'acide myristique et/ou de résidus d'acide gras du groupe constitué du résidu d'acides gras de l'acide caproïque, du résidu d'acides gras de l'acide caprylique, du résidu d'acides gras de l'acide caprique, du résidu d'acides gras de l'acide laurique et du résidu d'acides gras de l'acide myristique, est supérieure à 45 % en poids, de préférence supérieure à 55 % en poids, par rapport au poids total de la composition d'acides gras, et
dans lequel dans la composition d'acides gras utilisée à l'étape (A), la proportion de la quantité totale de
- l'acide laurique et/ou du résidu d'acide gras de l'acide laurique est supérieure à 20 % en poids, de préférence supérieure à 35 % en poids, de manière particulièrement préférée supérieure à 40 % en poids, de préférence, par rapport au poids total de la composition d'acides gras,
et/ou
- l'acide myristique et/ou du résidu d'acide gras de l'acide myristique est supérieure à 5 % en poids, de préférence supérieure à 10 % en poids, de manière particulièrement préférée supérieure à 15 % en poids, par rapport au poids total de la composition d'acide gras.

4. Procédé selon la revendication 3, dans lequel, à l'étape (A), le glycérol est utilisé, pour la réaction, en une quantité de 0,5 à 5 équivalents molaires, de préférence en une quantité de 1,0 à 2,0 équivalents molaires, de manière particulièrement préférée en une quantité de 1,25 à 1,75 équivalents molaires, par rapport à la quantité totale de composition d'acides gras,
et/ou
dans lequel la ou bien une, plusieurs ou toutes les base(s) catalytique(s) de l'étape (A) est / sont utilisée(s) en une quantité de 0,01 à 0,5 équivalents molaires, de préférence en une quantité de 0,025 à 0,1 équivalents molaires, de manière particulièrement préférée en une quantité de 0,05 à 0,1 équivalents molaires, par rapport à la quantité totale de composition d'acides gras
et/ou
dans lequel la ou bien une, plusieurs ou toutes les base(s) catalytique(s) de l'étape (A) est ou bien sont choisie(s) indépendamment les unes des autres dans le groupe constitué des bases aminés, des acides aminés, des bases de Brønsted, des bases de Lewis, des sels de métaux alcalins, des sels de métaux alcalino-terreux et de leurs mélanges, de préférence dans le groupe constitué par l'hydroxyde de sodium, l'hydroxyde de potassium et leurs mélanges.

5. Procédé selon l'une quelconque des revendications 3 à 4, dans lequel l'étape (A) est réalisée à une température comprise entre 150 et 300 °C, de préférence comprise entre 175 et 275 °C, de manière particulièrement préférée comprise entre 200 et 250 °C,
et en option dans des conditions de vide
et/ou
dans lequel, à l'étape (B), une quantité totale d'acide citrique et/ou d'un ou de plusieur(s) sel(s) de celui-ci est ajoutée dans une plage comprise entre 0,1 et 2,0 parties en poids, de préférence entre 0,25 et 1,5 partie(s) en poids, de manière particulièrement préférée comprise entre 0,25 et 1,0 partie en poids, par rapport au poids total du mélange obtenu à l'étape (A).

6. Procédé selon l'une quelconque des revendications 3 à 5, dans lequel, pour la réaction ayant lieu à l'étape (B) après addition d'acide citrique et/ou d'un ou de plusieurs sel(s) de celui-ci, une température comprise entre 50 et 200° C, de préférence comprise entre 100 et 175 °C, de manière particulièrement préférée comprise entre 120 et 160 °C
et en option des conditions de vide
est ou bien sont réglée(s).

7. Procédé selon l'une quelconque des revendications 3 à 6, dans lequel le ou bien un, plusieurs ou tous les solvant(s) de l'étape (C), si elle est présente, est ou bien sont de préférence choisi(s) dans le groupe constitué du pentane, de l'hexane, de l'heptane, du cyclopentane, du cyclohexane, du tétrachlorométhane, de l'éther diisopropylique, du toluène, du benzène, de l'éther diéthylique, du dichlorométhane, du chloroforme, de l'acétone, du dioxane, du tétrahydrofurane, du 2-méthylthétrahydrofurane, du tert-butylméthyléther, de l'acétate d'éthyle, du diméthylsulfoxyde, de l'acétonitrile, du benzonitrile, de la pyridine, du 2-propanol, de l'éthanol, du méthanol, de l'acide acétique, de l'acide formique, de l'eau et de mélanges de ceux-ci,
et/ou
la composition d'acides gras étant choisie dans le groupe constitué de l'huile de babassu, de l'huile de palmiste, de l'huile de noix de coco, de l'huile de macauba, de l'huile de microalgues, de l'huile neutre et de leurs mélanges.

8. Précurseur d'émulsifiant, obtenu par ou pouvant être obtenu par purification du mélange issu de l'étape (B) selon l'une quelconque des revendications 3 à 7
en utilisant un solvant ou plusieurs solvants choisi(s) dans le groupe constitué par les solvants halogénés, polaires, protiques polaires, protiques non polaires, "verts" et leurs mélanges,
et/ou
en mettant en oeuvre un procédé de purification ou plusieurs procédés de purification choisi(s) dans le groupe constitué par la filtration, l'ultra- ou la nanofiltration, les techniques d'adsorption et d'absorption, l'extraction, la recristallisation et les méthodes de chromatographie et leurs combinaisons.

9. Émulsifiant selon l'une quelconque des revendications 1 à 2, obtenu par ou pouvant être obtenu par un procédé selon l'une quelconque des revendications 3 à 7, de préférence
obtenu par ou pouvant être obtenu par un procédé selon l'une quelconque des revendications 3 à 8,
dans lequel le procédé comprend l'étape (C),
et en option dans lequel la proportion de la quantité totale de composés de formule (I) et d'un ou de plusieurs sel(s) du ou des composé(s) de formule (I) avec exactement un résidu d'acide citrique (iii) lié au squelette glycérol par une liaison ester est supérieure à 50 % en poids, de préférence supérieure à 85 % en poids, par rapport au poids total de l'émulsifiant, et de préférence dans lequel le rapport des proportions de la quantité totale de composés de formule (I) et d'un ou de plusieurs sel(s) du ou des composé(s) de formule (I) avec exactement un résidu d'acide gras lié au squelette glycérol par une liaison ester à la quantité totale de composés de formule (I) et d'un ou de plusieurs sel(s) du ou des composé(s) de formule (I) avec exactement deux résidus d'acide gras liés au squelette glycérol par des liaisons ester se situe dans la plage allant de 1 : 1,5 à 1 : 3, de préférence dans la plage allant de 1 : 1,75 à 1 : 2, de manière particulièrement préférée dans la plage allant de 1 : 2 à 1 : 2,5, par rapport au poids de ces composés.

10. Utilisation d'un mélange de composé(s) de formule (I) tel que défini dans l'une quelconque des revendications 1 ou 2, comme émulsifiant.

11. Émulsion comprenant ou constituée de
- une phase huileuse contenant un ou plusieurs précurseur(s) d'émulsifiant selon la revendication 8 et/ou un ou plusieurs émulsifiant(s) selon l'une quelconque des revendications 1, 2 ou 9 ou un mélange de ceux-ci,
et
- une phase aqueuse,
comprenant de préférence en outre un ou plusieurs composé(s) différents pour réduire ou augmenter la viscosité de l'émulsion.

12. Utilisation d'une émulsion selon la revendication 11 dans une préparation destinée au nettoyage, une préparation cosmétique ou pharmaceutique, de préférence dermatologique, une préparation servant au plaisir ou à la nutrition ou dans un produit semi-fini pour la réalisation d'une telle préparation.

13. Préparation destinée au nettoyage, préparation cosmétique ou pharmaceutique, de préférence dermatologique, préparation servant au plaisir ou à la nutrition ou produit semi-fini, comprenant un ou plusieurs précurseur(s) d'émulsifiant selon la revendication 8 ou un ou plusieurs émulsifiant(s) selon l'une quelconque des revendications 1, 2 ou 9 ou une ou plusieurs émulsion(s) selon la revendication 11.

14. Procédé de fabrication d'une émulsion selon la revendication 11, comprenant ou consistant en les étapes suivantes:
- fournir
une phase huileuse contenant un précurseur d'émulsifiant selon la revendication 8 ou un émulsifiant selon l'une quelconque des revendications 1, 2 ou 9 ou un mélange de ceux-ci,
et
une phase aqueuse,
- en option: chauffer de manière séparée la phase huileuse et la phase aqueuse, de préférence jusqu'à une température comprise entre 40 et 100 °C, de préférence comprise entre 60 et 85 °C,
- en option: ajouter à la phase huile chauffée un ou plusieurs composés différents pour réduire ou augmenter la viscosité,
- mélanger la phase aqueuse optionnellement chauffée et la phase huileuse chauffée, et
- en option: laisser refroidir en remuant.
